# EUROPEAN PATENT APPLICATION

(11) **EP 3 868 785 A1**
(43) Date of publication of application: **25.08.2021**
(21) Application number: 21150940.1
(22) Date of filing: 15.12.2016
(51) Int. Cl.: C07K 16/28, A61P 37/06

(54) **ANTI-CD28 HUMANIZED ANTIBODIES FORMULATED FOR ADMINISTRATION TO HUMANS**

(30) Priority: 15.12.2015 EP 15200281; 22.11.2016 EP 16306537
(62) Divisional of application: 16822423.6
(71) Applicant: OSE Immunotherapeutics, 44200 Nantes (FR)
(72) Inventor: VANHOVE, Bernard, 44400 Rézé (FR)
(74) Representative: Duffield, Stephen

(57) **Abstract**

The present invention pertains to a novel and advantageous dosage regimen for a humanized pegylated monovalent anti-CD28 Fab' antibody fragment, called "FR104". This dosage regimen consists of between 0.05 and 1.5 mg/kg body weight of FR104, at a dosing schedule of once per week, once every two weeks, once every three weeks, once every four weeks, once every five weeks or once every 6 weeks, once every 7 weeks, once every 8 weeks or once every more than 8 weeks.

## Description

### FIELD OF THE INVENTION

The present invention pertains to the field of immunotherapy. More specifically, the present invention provides a novel and advantageous dosage regimen for a humanized pegylated anti-CD28 Fab' antibody fragment previously described as "FR104" (Poirier et al., 2012).

### BACKGROUND AND PRIOR ART

CD28 is a dominant T cell positive costimulatory molecule. FR104 is a humanized monovalent anti-CD28 Fab' antibody fragment pegylated on the C-Terminal end of the heavy chain (WO 2011/101791, US 8,785,604 B2). FR104 originates from the humanization of a murine antibody called CD28.3. This antibody has been selected on the basis of its potent antagonist activity against CD80 binding to CD28 in Fab' format (Vanhove et al., 2003) and to its inherent inability to induce antigen-independent T cell activation (a process called superagonism), due to its target epitope lying outside the C"D antigenic loop of CD28, shown to be mandatory for superagonism (Poirier et al., 2012a).

FR104 binds to human and non-human primate CD28 (but not to mouse, rat, rabbit nor dog CD28) and antagonizes the binding of CD28 to its co-receptors CD80/CD86. The CD80/CD86 co-receptors therefore remain free to interact with the dominant negative costimulators Cytotoxic T-lymphocyte antigen 4 (CTLA-4) and programmed cell death 1 ligand 1 (PDL-1), which inhibit effector T cells and are mandatory for the suppressive function of regulatory T cells. The mechanism of action of FR104 is therefore double: 1) in a direct manner, it blocks CD28-mediated signals in T cells and 2), in an indirect manner, it promotes CTLA-4 and PDL-1-mediated signals. This mechanism of action is different from existing therapies aimed at blocking T cell costimulation such as abatacept (Orencia®) or belatacept (Nulojix®) that bind CD80 and CD86 and block function of both positive and negative costimulatory molecules (by blocking access to CD28, CTLA-4 and PDL-1).

Due to its monovalent nature, absence of an Fc domain and to the target epitope lying outside the target epitope of superagonist antibodies (such as TGN1412), FR104 is an antagonist antibody which cannot stimulate human T cells, even in the presence of antidrug antibodies (Poirier *et al.,* 2012). Available preclinical data demonstrate the absence of agonist and superagonist properties on human T cells *in vitro* and *trans-vivo,* in humanized mice, as well as *in vivo* in baboons, a species where, like for humans, T cells do release cytokines after interaction with superagonist anti-CD28 antibodies (Poirier *et al.,* 2014).

FR104 stayed antagonist in all these situations. No proliferation or cytokines secretion could be observed even if FR104 was coated, cross-linked with secondary antibodies or in the presence of anti-CD3 antibodies (Poirier *et al.,* 2012). Conversely, FR104 dosedependently inhibited T cell proliferation when human PBMC were stimulated by anti-CD3 antibody. In these assays, positive control superagonist anti-CD28 antibodies consistently induced human T cell proliferation and/or cytokine release.

Due to its selective immunosuppressive activity directed at effector T cells, FR104 is first investigated for use in rheumatoid arthritis and in transplantation (kidney transplant recipients and graft-versus-host disease [GVHD] after stem cell transplantation). There is a direct expected benefit of FR104 for patients with dysregulated self or allogeneic immune responses consisting in an improved efficacy of T cell immunosuppression and in a long-term control of pathological T and B cell responses due to the preservation of immune regulatory mechanisms. For kidney transplant patients, this should reduce early rejection events and improve long-term outcomes. For patients suffering from a moderate-to-severe GVHD after allogeneic hematopoietic stem cells transplantation, this should blunt alloreactivity of effector T cells. For patients suffering from rheumatoid arthritis, this should translate into a rapid improvement of clinical symptoms and less frequent relapses. Available preclinical evaluations include demonstration of efficacy in kidney transplantation in primates (Poirier *et al.,* 2015) and in GVHD in humanized mice (Poirier *et al.,* 2012) and primates (Kean *et al.,* 2014), in experimental models of autoimmune encephalomyelitis (Haanstra et al, JI 2015), skin inflammation in primate model of human psoriasis (Poirier et al, JI 2015) (Poirier et al, Experimental Dermatol. 2015) and a collagen induced arthritis in primate, model of rheumatoid arthritis (Vierboom *et al.,* 2015).

Mechanistically, it has been demonstrated that FR104 blocks alloreactivity in a CTLA-4 dependent manner, which confirms that the theoretical mechanism of action is indeed operating *in vivo* (Poirier *et al.,* 2012).

FR104 is produced by classical recombinant technology in Chinese Hamster Ovary (CHO) cells. It is a humanized pegylated Fab' antibody fragment consisting of (i) a first protein of SEQ ID NO: 1, which is pegylated at its C-terminus, and (ii) a second protein of SEQ ID NO: 2. After purification, it is chemically bound to a poly-ethylene glycol moiety (PEG) to increase its plasma half-life. The C-terminus of the CH1 domain has been engineered to accommodate a monopegylation. The PEG residue is a bi-branched 2x20 kDa linked to the C-terminal cysteine via a maleimide ring. All the production, purification and vialing processes are performed under GMP conditions.

Poirier et al (2012), showed by plasmon resonance analysis, that FR104 dissociation constant to CD28 is about 4.6nM. Calculation of the minimum anticipated biological effect level (MABEL) and dose selection for the first-in-human (FIH) clinical trial with FR104 were based on the data published by Poirier et al. (2012).

### SUMMARY OF THE INVENTION

As described in the experimental part below, the data obtained by the first experiments of the clinical trial were very surprising and led the inventors to the conclusion that the real K_{D} value is at least 10-fold lower than the value of 4.6nM measured *in vitro.*

The inventors, pursuing their investigations, reviewed the pharmacokinetic/target engagement (PK/TE) model to capture the obtained PK and receptor occupancy (RO) data. Based on this revised model:
- 0.05 mg/kg dosing may achieve a mean RO% >80% for 2-3 weeks; and
- 0.2 mg/kg dosing may achieve a mean RO% >80% for 4-5 weeks.

The present invention hence pertains to the use of FR104 for treating any condition susceptible of being improved or prevented by inhibiting a T cell immune response, by administering a therapeutically effective amount of FR104 to a human subject in need thereof, wherein the therapeutically effective amount of FR104 is between 0.05 and 1.5 mg/kg body weight at a dosing schedule of once per week, once every two weeks, once every three weeks, once every four weeks, once every five weeks or once every 6 weeks. These administrations can be performed during long periods (several months to several years) or stopped after one or a few administrations. They can also be interrupted during a while (a few months or years) and renewed if the patient's condition justifies a new treatment.

The invention provides an anti-CD28 Fab' antibody fragment consisting of a heterodimer of (i) a first protein of SEQ ID NO: 1, which is pegylated at its C-terminus, and (ii) a second protein of SEQ ID NO: 2, for use in the treatment of any condition susceptible of being improved or prevented by inhibiting a T cell immune response, wherein a therapeutically effective amount of said anti-CD28 Fab' antibody fragment is administered to a subject in need thereof and wherein the therapeutically effective amount of the anti-CD28 Fab' antibody fragment is between 0.05 and 1.5 mg/kg body weight at a dosing schedule of once per week, once every two weeks, once every three weeks, once every four weeks, once every five weeks or once every 6 weeks, once every 7 weeks, once every 8 weeks or once every more than 8 weeks. In some embodiments the therapeutically effective amount is between 0.05 and less than 0.5 mg/kg body weight, administered at a dosing schedule of once per week, once every two weeks, once every three weeks, once every four weeks, once every five weeks or once every 6 weeks. In some embodiments, the therapeutically effective amount is between 0.05 and 0.2 mg/kg body weight administered at a dosing schedule of once per week, once every two weeks, once every three weeks, once every four weeks or once every five weeks. In some embodiments, the therapeutically effective amount is between 0.5 and 1.5 mg/kg body weight, administered at a dosing schedule of from once every at least four weeks for 0.5 mg/kg once every at least 8 weeks for 1 mg/kg and once every more than 8 weeks for doses above 1 mg/kg. In some embodiments, the therapeutically effective amount of an anti-CD28 Fab' antibody fragment induces at least 80% CD28 receptor occupancy over the period of time between two administrations of said anti-CD28 Fab' antibody fragment. In some embodiments, the condition susceptible of being improved or prevented by inhibiting a T cell immune response is a transplanted organ, tissue or cell dysfunction, a T-lymphocyte-mediated autoimmune disease, atherosclerosis or an inflammatory disease in said subject. In some embodiments, the condition susceptible of being improved or prevented by inhibiting a T cell immune response is a kidney transplant rejection, a chronic allograft vasculopathy, a graft-versus-host disease, an autoimmune encephalomyelitis, a psoriasis, a rheumatoid arthritis, a multiple sclerosis, a Crohn's disease, an ulcerative colitis, atherosclerosis, a type 1 diabetes or a type IV hypersensitivity. In some embodiments, the anti-CD28 Fab' antibody fragment is administered to said subject intravenously, subcutaneously, intramuscularly, or topically via intrathecal injection. Another aspect of the present invention is a pharmaceutical composition for inhibiting a T cell immune response in a human subject in need thereof, comprising FR104 in an amount comprised between 3 and 120 mg, preferably less than 35 mg per dose, together with one or more pharmaceutically acceptable excipients. In some embodiments, the pharmaceutical composition comprises between 3 and 14 mg of said anti-CD28 Fab' antibody fragment. In some embodiments, the pharmaceutical composition is suitable for intravenous, subcutaneous, intramuscular, topical or intrathecal administration. In some embodiments, the pharmaceutical composition comprises recombinant human hyaluronidase.

A syringe comprising the above- described pharmaceutical composition is part of the present invention as well. The syringe may be adapted for subcutaneous use.

The present invention also pertains to a kit of parts comprising monthly doses of FR104, wherein each dose comprises an amount of 3 to 120 mg of FR104.

### LEGENDS TO THE FIGURES

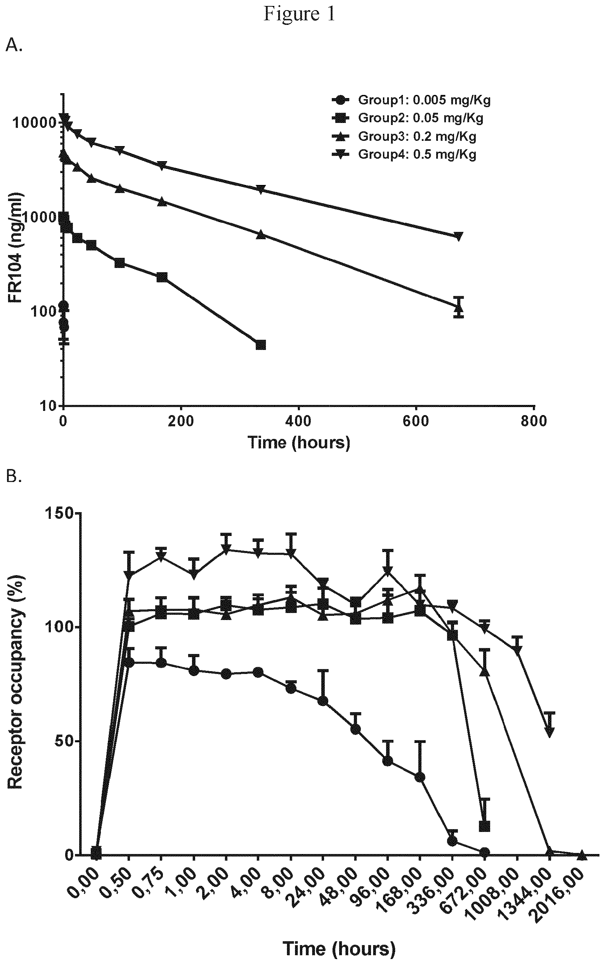

Figure 1: FR104 Pharmacokinetic study and CD28 Receptor occupancy. A. FR104 concentration time course in the serum of volunteers receiving different concentrations of FR104 as follows: Group1 (black circle) received 0.005mg/kg, Group2 (black square) received 0.05mg/kg, Group 3 (up black triangle) 0.2mg/kg and Group4 (down black triangle) 0.5mg/kg. **B.** Time course of the receptor occupancy in each volunteer in each group receiving FR104 (as referred above).

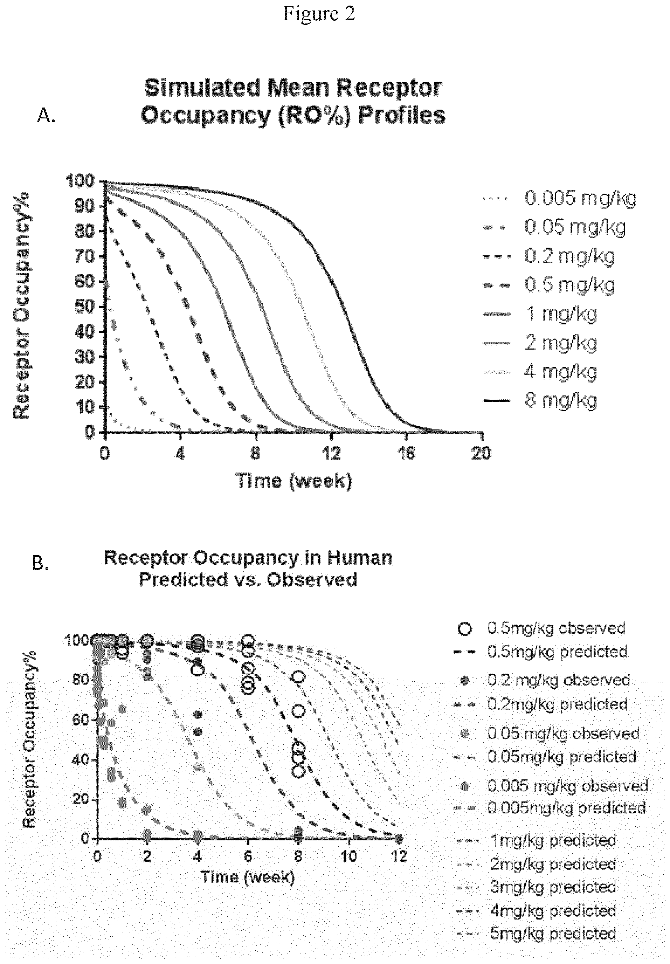

Figure 2: CD28 receptor occupancy predicted versus observed. A. Percentage of the RO previously predicted over a time course of 20 weeks for each concentration of FR104 determined with the *in vitro* calculated Kd. **B.** Time course of the modified predicted percentage of the RO based on the recalculated Kd compared with the observed percentage of the RO.

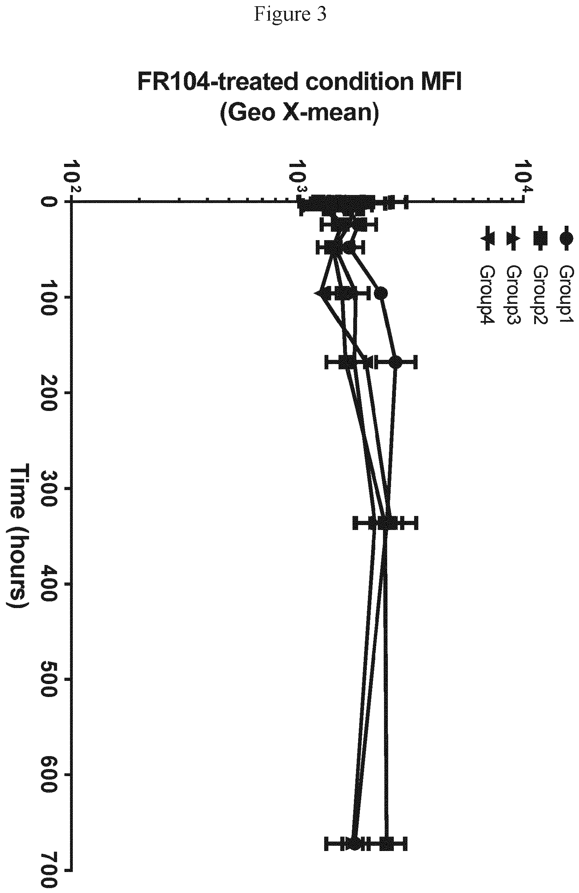

Figure 3: Receptor expression post-infusion of FR104. In each group receiving FR104, the CD28 expression in the serum of volunteers was analyzed by flow cytometry over a time course. Group1 (black circle) received 0.005mg/kg, Group2 (black square) received 0.05 mg/kg, Group3 (up triangle) received 0.2 mg/kg and Group 4 (down triangle) received 0.5 mg/kg.

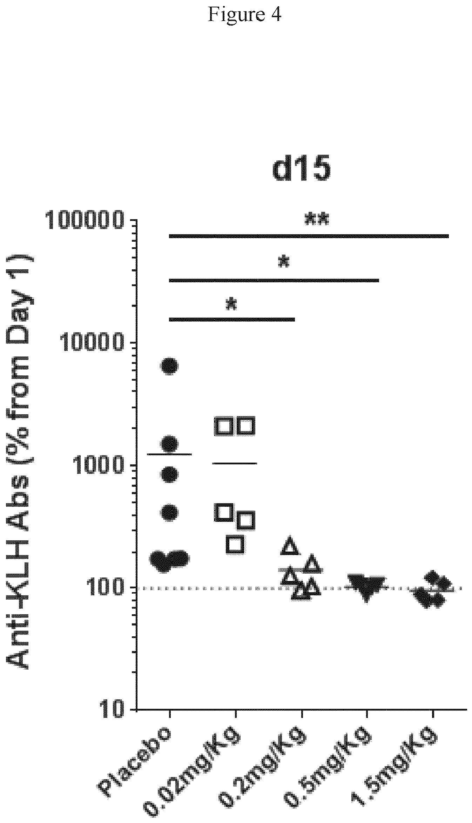

Figure 4: anti-KLH (IgG) antibody measurement. Percentage of the anti-KLH antibody present in serum of volunteers 15 days after the first infusion in five different groups receiving: with placebo, with FR104 at 0.02mg/kg, 0.2mg/kg, 0.5mg/kg or at 1.5 mg/kg. This percentage represents the ratio between the antibody concentration in the serum at Day1 (first infusion) and at Day 15.

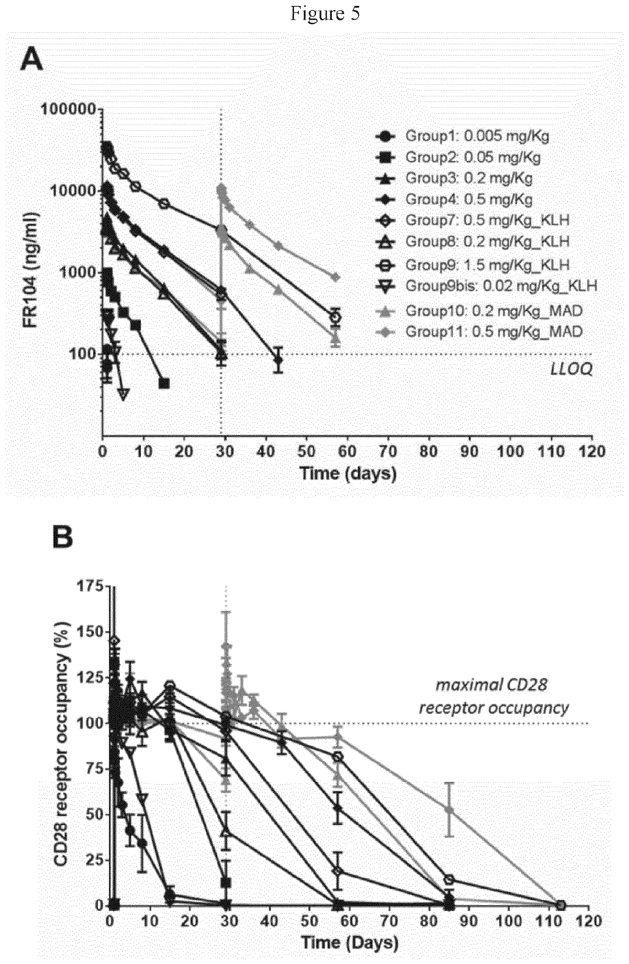

Figure 5: Pharmacokinetic and pharmacodynamic data. A. Mean serum FR104 concentrations ± SEM (semi-log scale) from pre-dose to end of study. Treatment groups shown are: single ascending doses (black symbols), single ascending doses + KLH immunisation (empty symbols), multiple ascending doses (MAD, shaded symbols). LLOQ: Lower limit of Quantification. N = 3-5 subjects per treatment group. B. Mean CD28 receptor occupancy ± SEM across the FR104 treatment groups indicated in A from predose to end of study. N = 3-5 subjects per treatment group. Assessment of receptor occupancy in subjects dosed with placebo resulted in a background signal ranging between 0 and 5%.

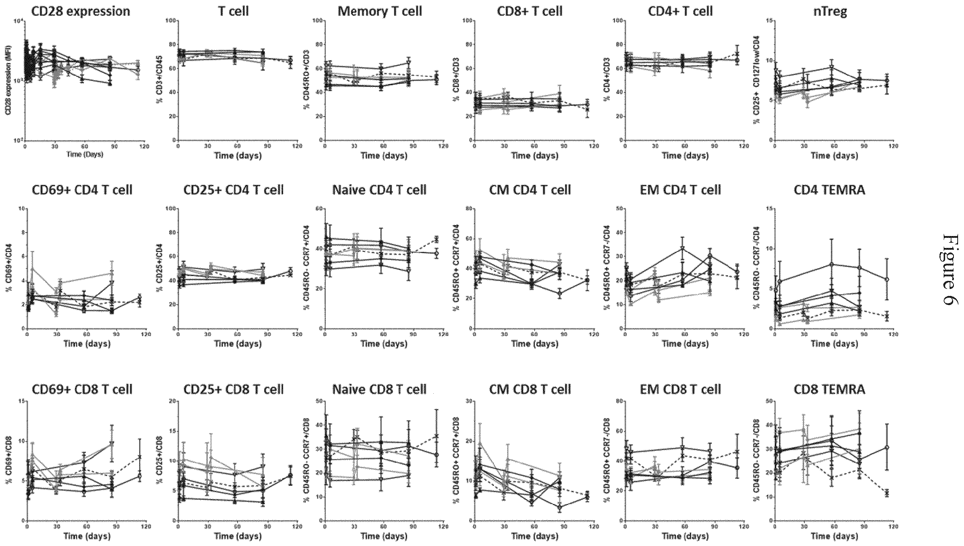

Figure 6: Flow cytometry analysis of CD28 expression level, T lymphocyte sub-populations frequency and activated T-cells frequency on stabilized whole blood samples. T-cell sub-populations were defined using the following gating strategy in CD45+ CD3+ cells: natural Treg (nTreg): CD25+ CD127 low CD4+; activated: CD69+ or CD25+; memory T cells: CD45RO+; naive: CD45RO- CCR7+; central memory (CM): CD45RO+ CCR7+; effector memory (EM): CD45RO+ CCR7-; TEMRA: CD45RO- CCR7-. Symbols refer to the groups defined in Figure 1A, plus 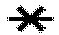 for pooled placebo subjects (n=12). Data are means ± SEM.

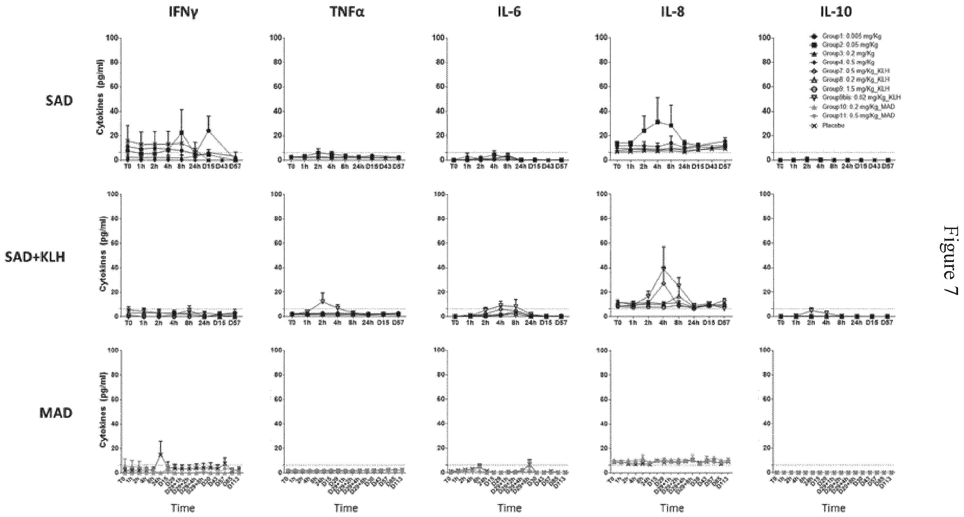

Figure 7: Serum IFN-γ, TNF-α, IL-6, IL-8, IL-10 cytokines in FR104 and placebo recipients from predose to the end of the study. Dotted bar: lower limit of quantification. Data are means ± SEM. SAD: single ascending doses. SAD + KLH, single ascending dosed subjects Immunized with KLH. MAD: multiple ascending doses. Symbols refer to the groups defined in Figure 1A, plus 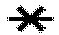 for placebo subjects in SAD (n=6), SAD + KLH (n=8) and MAD (n=4) groups. Other cytokines assessed (IL 12p70 IL-1β, IL-2, IL-4) were found negative for all samplings (not shown).

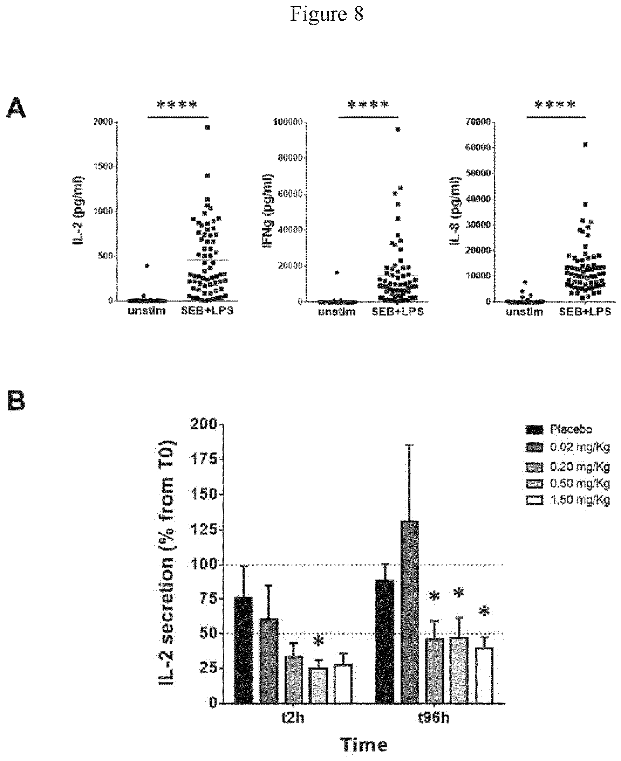

Figure 8: Ex-vivo stimulation of blood cells with SEB + LPS in whole-blood cultures (TruCulture®). A. Blood samples at baseline were not stimulated or stimulated with SEB + LPS and maintained for 24h in culture. Cytokines in the plasma were then assessed by ELISA. Dots represent individual subjects. B. Measurement of IL-2 secretion by SEB + LPS stimulated blood samples drawn at the indicated time points in single ascending dose groups (with or without KLH stimulation) receiving FR104 at the indicated dose level. Only cultures of the donors tested in this study showing sufficient responses to the stimuli at baseline (>100pg/ml) have been included in the analysis: Placebo (n=9), 0.02 mg/Kg (n=5), 0.20 mg/Kg (n=9), 0.50 mg/Kg (n=9) and 1.50 mg/Kg (n=3) Data are mean IL-2 concentrations normalized to T0 time points, ± SEM. *: p<0.05; ****: p<0.0001.

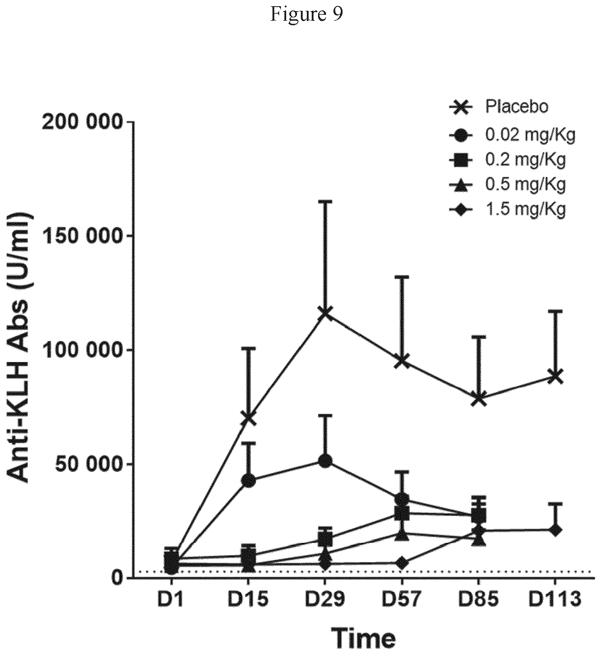

Figure 9: Anti-KLH IgG (ng/mL) levels over time by treatment group. Serum samples were drawn at the indicated time points and assessed by anti-KLH ELISA. Data are means ± SEM. Dotted line: lower limit of quantification.

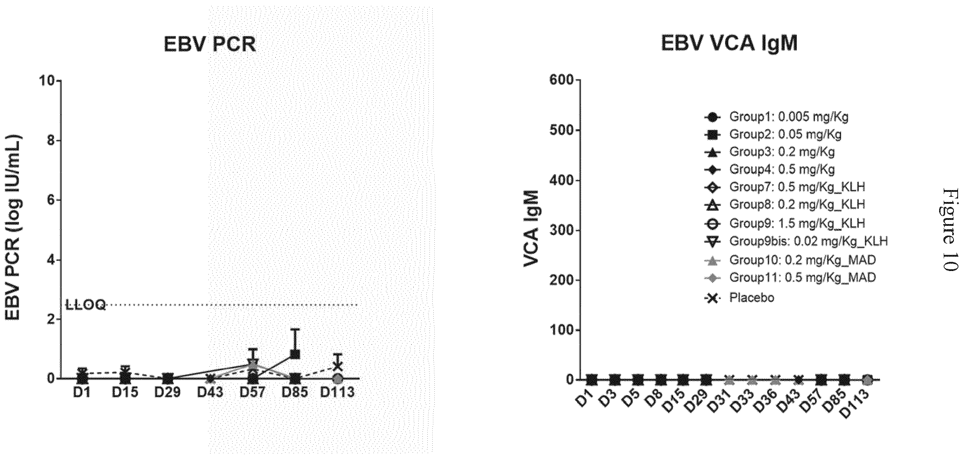

Figure 10: Follow of the EBV status from pre-dose to end of study. EBV viral load was assessed in blood by PCR at the indicated timepoints. IgM antibodies against EBV capsid antigen (VCA) were also assessed in serum to measure anti-EBV immune reactivation. Data are means ± SEM. LLOQ: Lower limit of quantification.

### DETAILED DESCRIPTION OF THE PREFERRED EMBODIMENTS

Throughout the present text, the following definitions are used:

### FR104

"FR104" designates an anti-CD28 Fab' antibody fragment consisting of a heterodimer of (i) a first protein of SEQ ID NO: 1, which is pegylated at its C-terminus, and (ii) a second protein of SEQ ID NO: 2. According to a particular embodiment, FR104 is an anti-CD28 Fab' antibody fragment consisting of a heterodimer of (i) a first protein of SEQ ID NO: 3, which is pegylated at its C-terminus, and (ii) a second protein of SEQ ID NO: 4. For example, a bi-branched 2x20 kDa PEG can be linked to the C-terminal cysteine of the CH1 domain via a maleimide ring.

### Treatment

As used herein, the terms "treat", "treatment" and "treating" refer to any reduction or amelioration of the progression, severity, and/or duration of a disease. For example, in a pathology such as rheumatoid arthritis, reduction of one or more symptoms thereof is considered as a treatment.

Other definitions will be specified below, when necessary.

A first aspect of the present invention is the use of FR104, in the treatment of any condition susceptible of being improved or prevented by inhibiting a T cell immune response, wherein a therapeutically effective amount of said anti-CD28 Fab' antibody fragment is administered to a human subject in need thereof and wherein the therapeutically effective amount of the anti-CD28 Fab' antibody fragment is between 0.05 and 1.5 mg/kg body weight at a dosing schedule of once per week, once every two weeks, once every three weeks, once every four weeks, once every five weeks, once every 6 weeks, once every 7 weeks, once every 8 weeks, once every 9 weeks or once every more than 9 weeks.

A method of inhibiting a T cell immune response in a human subject in need thereof is also part of the present invention. According to this method, a therapeutically effective amount of FR104 is administered to the subject, wherein said therapeutically effective amount is between 0.05 and 1.5 mg/kg body weight, administered at a dosing schedule of once per week, once every two weeks, once every three weeks, once every four weeks, once every five weeks, once every 6 weeks, once every 7 weeks, once every 8 weeks, once every 9 weeks or once every more than 9 weeks.

As mentioned above, the treatment according to the present invention can be administered during long periods of time (several months to several years) or stopped after one or a few administrations, depending on the patient's condition. The treatment can also be interrupted during a while (a few months or years) and renewed if the patient's condition justifies additional administrations of FR104. According to a particular embodiment of the invention, the therapeutically effective amount is between 0.05 and less than 0.5 mg/kg body weight, administered at a dosing schedule of once per week, once every two weeks, once every three weeks, once every four weeks, once every five weeks or once every 6 weeks. In some embodiments, the therapeutically effective amount is between 0.05 and 0.5 mg/kg body weight, administered at a dosing schedule of once per week, such as at 0.1 - 0.45 mg/kg, 0.15 - 0.4mg/kg, 0.2 - 0.35 mg/kg, 0.25 - 0.3 mg/kg, 0.05 - 0.4 mg/kg, 0.05 - 0.3 mg/kg, 0.1 - 0.4 mg/kg, 0.1 - 0.3 mg/kg, 0.5 mg/kg (optionally ± 10%), 0.4 mg/kg (optionally ± 10%), 0.3 mg/kg (optionally ± 10%) or 0.25 mg/kg (optionally ± 10%). In some embodiments, the therapeutically effective amount is between 0.05 and 0.5 mg/kg body weight, administered at a dosing schedule of once every two weeks, such as at 0.1 - 0.45 mg/kg, 0.15 - 0.4mg/kg, 0.2 - 0.35 mg/kg, 0.25 - 0.3 mg/kg, 0.05 - 0.4 mg/kg, 0.05 - 0.3 mg/kg, 0.1 - 0.4 mg/kg, 0.1 - 0.3 mg/kg, 0.5 mg/kg (optionally ± 10%), 0.4 mg/kg (optionally ± 10%), 0.3 mg/kg (optionally ± 10%) or 0.25 mg/kg (optionally ± 10%). In some embodiments, the therapeutically effective amount is between 0.05 and 0.5 mg/kg body weight, administered at a dosing schedule of once every three weeks, such as at 0.1 - 0.45 mg/kg, 0.15 - 0.4mg/kg, 0.2 - 0.35 mg/kg, 0.25 - 0.3 mg/kg, 0.05 - 0.4 mg/kg, 0.05 - 0.3 mg/kg, 0.1 - 0.4 mg/kg, 0.1 - 0.3 mg/kg, 0.5 mg/kg (optionally ± 10%), 0.4 mg/kg (optionally ± 10%), 0.3 mg/kg (optionally ± 10%) or 0.25 mg/kg (optionally ± 10%). In some embodiments, the therapeutically effective amount is between 0.05 and 0.5 mg/kg body weight, administered at a dosing schedule of once every four weeks, such as at 0.1 - 0.45 mg/kg, 0.15 - 0.4mg/kg, 0.2 - 0.35 mg/kg, 0.25 - 0.3 mg/kg, 0.05 - 0.4 mg/kg, 0.05 - 0.3 mg/kg, 0.1 - 0.4 mg/kg, 0.1 - 0.3 mg/kg, 0.5 mg/kg (optionally ± 10%), 0.4 mg/kg (optionally ± 10%), 0.3 mg/kg (optionally ± 10%) or 0.25 mg/kg (optionally ± 10%). In some embodiments, the therapeutically effective amount is between 0.05 and 0.5 mg/kg body weight, administered at a dosing schedule of once every five weeks, such as at 0.1 - 0.45 mg/kg, 0.15 - 0.4mg/kg, 0.2 - 0.35 mg/kg, 0.25 - 0.3 mg/kg, 0.05 - 0.4 mg/kg, 0.05 - 0.3 mg/kg, 0.1 - 0.4 mg/kg, 0.1 - 0.3 mg/kg, 0.5 mg/kg (optionally ± 10%), 0.4 mg/kg (optionally ± 10%), 0.3 mg/kg (optionally ± 10%) or 0.25 mg/kg (optionally ± 10%). In some embodiments, the therapeutically effective amount is between 0.05 and 0.5 mg/kg body weight, administered at a dosing schedule of once every six weeks, such as at 0.1 - 0.45 mg/kg, 0.15 - 0.4mg/kg, 0.2 - 0.35 mg/kg, 0.25 - 0.3 mg/kg, 0.05 - 0.4 mg/kg, 0.05 - 0.3 mg/kg, 0.1 - 0.4 mg/kg, 0.1 - 0.3 mg/kg, 0.5 mg/kg (optionally ± 10%), 0.4 mg/kg (optionally ± 10%), 0.3 mg/kg (optionally ± 10%) or 0.25 mg/kg (optionally ± 10%).

According to another particular embodiment of the invention, the therapeutically effective amount is between 0.05 and 0.2 mg/kg body weight, administered at a dosing schedule of once per week, once every two weeks, once every three weeks ,once every four weeks or once every five weeks. In some embodiments, the therapeutically effective amount is between 0.05 and 0.2 mg/kg body weight, administered at a dosing schedule of once per week, such as at 0.05 - 0.15 mg/kg, 0.1 - 0.2mg/kg, 0.1 - 0.15 mg/kg, 0.05 mg/kg (optionally ± 10%), 0.1 mg/kg (optionally ± 10%), 0.15 mg/kg (optionally ± 10%) or 0.2 mg/kg (optionally ± 10%). In some embodiments, the therapeutically effective amount is between 0.05 and 0.2 mg/kg body weight, administered at a dosing schedule of once every two weeks, such as at 0.05 - 0.15 mg/kg, 0.1 - 0.2mg/kg, 0.1 - 0.15 mg/kg, 0.05 mg/kg (optionally ± 10%), 0.1 mg/kg (optionally ± 10%), 0.15 mg/kg (optionally ± 10%) or 0.2 mg/kg (optionally ± 10%). In some embodiments, the therapeutically effective amount is between 0.05 and 0.2 mg/kg body weight, administered at a dosing schedule of once every three weeks, such as at 0.05 - 0.15 mg/kg, 0.1 - 0.2mg/kg, 0.1- 0.15 mg/kg, 0.05 mg/kg (optionally ± 10%), 0.1 mg/kg (optionally ± 10%), 0.15 mg/kg (optionally ± 10%) or 0.2 mg/kg (optionally ± 10%). In some embodiments, the therapeutically effective amount is between 0.05 and 0.2 mg/kg body weight, administered at a dosing schedule of once every four weeks, such as at 0.05 - 0.15 mg/kg, 0.1 - 0.2mg/kg, 0.1 - 0.15 mg/kg, 0.05 mg/kg (optionally ± 10%), 0.1 mg/kg (optionally ± 10%), 0.15 mg/kg (optionally ± 10%) or 0.2 mg/kg (optionally ± 10%). In some embodiments, the therapeutically effective amount is between 0.05 and 0.2 mg/kg body weight, administered at a dosing schedule of once every five weeks, such as at 0.05 - 0.15 mg/kg, 0.1 - 0.2mg/kg, 0.1 - 0.15 mg/kg, 0.05 mg/kg (optionally ± 10%), 0.1 mg/kg (optionally ± 10%), 0.15 mg/kg (optionally ± 10%) or 0.2 mg/kg (optionally ± 10%).

When an amount of between 0.5 and 1.5 mg/kg body weight is administered, the frequency of medication uptake is preferably reduced. With such doses, administration of one dose per month or even less frequently is advantageously prescribed. According to yet another particular embodiment of the invention, the therapeutically effective amount is between 0.5 and 1.5 mg/kg body weight, administered at a dosing schedule of once every three weeks, once every four weeks, once every five weeks, once every 6 weeks, once every 7 weeks, once every 8 weeks, once every 9 weeks or once every more than 9 weeks. In some embodiments, the therapeutically effective amount is between 0.5 and 1.5 mg/kg body weight, administered at a dosing schedule of once every four weeks, such as at 0.6 - 1.4 mg/kg, 0.7 - 1.3mg/kg, 0.8 - 1.2 mg/kg, 0.9 - 1.1 mg/kg, 0.5 - 1.4 mg/kg, 0.5 - 1.3 mg/kg, 0.5 - 1.2 mg/kg, , 0.5 - 1.1 mg/kg, 0.5 - 1.0 mg/kg, 0.5 - 0.9 mg/kg, 0.5 - 0.8 mg/kg, 0.5 - 0.7 mg/kg, 0.5 - 0.6 mg/kg, 0.5 mg/kg (optionally ± 10%), 0.6 mg/kg (optionally ± 10%), 0.7 mg/kg (optionally ± 10%), 0.8 mg/kg (optionally ± 10%), 0.9 mg/kg (optionally ± 10%), 1.0 mg/kg (optionally ± 10%), 1.1 mg/kg (optionally ± 10%), 1.2 mg/kg (optionally ± 10%), 1.3 mg/kg (optionally ± 10%), 1.4 mg/kg (optionally ± 10%) or 1.5 mg/kg (optionally ± 10%). In some embodiments, the therapeutically effective amount is between 0.5 and 1.5 mg/kg body weight, administered at a dosing schedule of once every five weeks, such as at 0.6 - 1.4 mg/kg, 0.7 - 1.3mg/kg, 0.8 - 1.2 mg/kg, 0.9 -1.1 mg/kg, 0.5 - 1.4 mg/kg, 0.5 - 1.3 mg/kg, 0.5 - 1.2 mg/kg, , 0.5 -1.1 mg/kg, 0.5 - 1.0 mg/kg, 0.5 - 0.9 mg/kg, 0.5 - 0.8 mg/kg, 0.5 - 0.7 mg/kg, 0.5 - 0.6 mg/kg, 0.5 mg/kg (optionally ± 10%), 0.6 mg/kg (optionally ± 10%), 0.7 mg/kg (optionally ± 10%), 0.8 mg/kg (optionally ± 10%), 0.9 mg/kg (optionally ± 10%), 1.0 mg/kg (optionally ± 10%), 1.1 mg/kg (optionally ± 10%), 1.2 mg/kg (optionally ± 10%), 1.3 mg/kg (optionally ± 10%), 1.4 mg/kg (optionally ± 10%) or 1.5 mg/kg (optionally ± 10%). In some embodiments, the therapeutically effective amount is between 0.5 and 1.5 mg/kg body weight, administered at a dosing schedule of once every six weeks, such as at 0.6 - 1.4 mg/kg, 0.7 - 1.3mg/kg, 0.8 - 1.2 mg/kg, 0.9 - 1.1 mg/kg, 0.5 - 1.4 mg/kg, 0.5 - 1.3 mg/kg, 0.5 - 1.2 mg/kg, , 0.5 - 1.1 mg/kg, 0.5 - 1.0 mg/kg, 0.5 - 0.9 mg/kg, 0.5 - 0.8 mg/kg, 0.5 - 0.7 mg/kg, 0.5 - 0.6 mg/kg, 0.5 mg/kg (optionally ± 10%), 0.6 mg/kg (optionally ± 10%), 0.7 mg/kg (optionally ± 10%), 0.8 mg/kg (optionally ± 10%), 0.9 mg/kg (optionally ± 10%), 1.0 mg/kg (optionally ± 10%), 1.1 mg/kg (optionally ± 10%), 1.2 mg/kg (optionally ± 10%), 1.3 mg/kg (optionally ± 10%), 1.4 mg/kg (optionally ± 10%) or 1.5 mg/kg (optionally ± 10%). In some embodiments, the therapeutically effective amount is between 0.5 and 1.5 mg/kg body weight, administered at a dosing schedule of once every seven weeks, such as at 0.6 - 1.4 mg/kg, 0.7 - 1.3mg/kg, 0.8 - 1.2 mg/kg, 0.9 - 1.1 mg/kg, 0.5 - 1.4 mg/kg, 0.5 - 1.3 mg/kg, 0.5 - 1.2 mg/kg, , 0.5 - 1.1 mg/kg, 0.5 - 1.0 mg/kg, 0.5 - 0.9 mg/kg, 0.5 - 0.8 mg/kg, 0.5 - 0.7 mg/kg, 0.5 - 0.6 mg/kg, 0.5 mg/kg (optionally ± 10%), 0.6 mg/kg (optionally ± 10%), 0.7 mg/kg (optionally ± 10%), 0.8 mg/kg (optionally ± 10%), 0.9 mg/kg (optionally ± 10%), 1.0 mg/kg (optionally ± 10%), 1.1 mg/kg (optionally ± 10%), 1.2 mg/kg (optionally ± 10%), 1.3 mg/kg (optionally ± 10%), 1.4 mg/kg (optionally ± 10%) or 1.5 mg/kg (optionally ± 10%). In some embodiments, the therapeutically effective amount is between 0.5 and 1.5 mg/kg body weight, administered at a dosing schedule of once every eight weeks, such as at 0.6 - 1.4 mg/kg, 0.7 - 1.3mg/kg, 0.8 - 1.2 mg/kg, 0.9 -1.1 mg/kg, 0.5 - 1.4 mg/kg, 0.5 - 1.3 mg/kg, 0.5 - 1.2 mg/kg, , 0.5 -1.1 mg/kg, 0.5 - 1.0 mg/kg, 0.5 - 0.9 mg/kg, 0.5 - 0.8 mg/kg, 0.5 - 0.7 mg/kg, 0.5 - 0.6 mg/kg, 0.5 mg/kg (optionally ± 10%), 0.6 mg/kg (optionally ± 10%), 0.7 mg/kg (optionally ± 10%), 0.8 mg/kg (optionally ± 10%), 0.9 mg/kg (optionally ± 10%), 1.0 mg/kg (optionally ± 10%), 1.1 mg/kg (optionally ± 10%), 1.2 mg/kg (optionally ± 10%), 1.3 mg/kg (optionally ± 10%), 1.4 mg/kg (optionally ± 10%) or 1.5 mg/kg (optionally ± 10%). In some embodiments, the therapeutically effective amount is between 0.5 and 1.5 mg/kg body weight, administered at a dosing schedule of once every nine weeks, such as at 0.6 - 1.4 mg/kg, 0.7 - 1.3mg/kg, 0.8 - 1.2 mg/kg, 0.9 -1.1 mg/kg, 0.5 - 1.4 mg/kg, 0.5 - 1.3 mg/kg, 0.5 - 1.2 mg/kg, , 0.5 - 1.1 mg/kg, 0.5 - 1.0 mg/kg, 0.5 - 0.9 mg/kg, 0.5 - 0.8 mg/kg, 0.5 - 0.7 mg/kg, 0.5 - 0.6 mg/kg, 0.5 mg/kg (optionally ± 10%), 0.6 mg/kg (optionally ± 10%), 0.7 mg/kg (optionally ± 10%), 0.8 mg/kg (optionally ± 10%), 0.9 mg/kg (optionally ± 10%), 1.0 mg/kg (optionally ± 10%), 1.1 mg/kg (optionally ± 10%), 1.2 mg/kg (optionally ± 10%), 1.3 mg/kg (optionally ± 10%), 1.4 mg/kg (optionally ± 10%) or 1.5 mg/kg (optionally ± 10%). In some embodiments, the therapeutically effective amount is between 0.5 and 1.5 mg/kg body weight, administered at a dosing schedule of once every more than nine weeks, such as at 0.6 - 1.4 mg/kg, 0.7 - 1.3mg/kg, 0.8 - 1.2 mg/kg, 0.9 -1.1 mg/kg, 0.5 - 1.4 mg/kg, 0.5 - 1.3 mg/kg, 0.5 - 1.2 mg/kg, , 0.5 - 1.1 mg/kg, 0.5 - 1.0 mg/kg, 0.5 - 0.9 mg/kg, 0.5 - 0.8 mg/kg, 0.5 - 0.7 mg/kg, 0.5 - 0.6 mg/kg, 0.5 mg/kg (optionally ± 10%), 0.6 mg/kg (optionally ± 10%), 0.7 mg/kg (optionally ± 10%), 0.8 mg/kg (optionally ± 10%), 0.9 mg/kg (optionally ± 10%), 1.0 mg/kg (optionally ± 10%), 1.1 mg/kg (optionally ± 10%), 1.2 mg/kg (optionally ± 10%), 1.3 mg/kg (optionally ± 10%), 1.4 mg/kg (optionally ± 10%) or 1.5 mg/kg (optionally ± 10%).

It is important to note that the therapeutic doses envisioned from *in vitro* data were between 0.5 mg/kg and 8 mg/kg. As disclosed in the experimental part below, the data obtained from the first experiments of the first-in-human phase I study did not concord with the pharmacokinetic/target engagement (PK/TE) model calculated on the basis of the data that were available before the beginning of this first-in-human study. The *in vivo* data showed that an effect is observed with doses as low as 0.05 mg/kg, which was completely unexpected. This lead the inventors to modify the pharmacokinetic/target engagement (PK/TE) model and to reconsider the affinity of FR104 for human CD28, with a K_{D} value of around 0.3 nM instead of 4.6 nM. As a result, the maximal dose which is now envisioned is 1.5 mg/kg, and doses as low as 0.05 mg/kg are considered as therapeutically affective.

In some embodiments, the dose of FR104 has a half life in humans between 120 and 240 hours, such as between 130 and 230 hours, between 140 and 220 hours or between 150 and 210 hours.

In some embodiments, serum IFNg TNFa and IL-8 levels following administration of FR104 to a human subject are below 5000 pg/ml, such as below 4000 pg/ml, below 3000 pg/ml, below 2000 pg/ml, below 1000 pg/ml, below 800 pg/ml, below 600 pg/ml, below 400 pg/ml, or below 200 pg/ml.

The dosage regimen according to the present invention is thus drastically different from what was expected from the data available before the first-in-human study disclosed below. This change in dosage regimen has several advantages, such as a reduced cost of the treatment, reduced adverse side-effects, a reduction of the frequency of administration and less difficulties for formulating the pharmaceutical compositions comprising FR104 as an active principle. Indeed, it is not always possible or convenient to administer big volumes of a therapeutic solution (> 2 ml) to a patient, especially through the subcutaneous route.

It is currently thought that a receptor occupancy of at least 80% is necessary for obtaining a therapeutically effective treatment with FR104. According to a preferred embodiment of the present invention, the amount of FR104 administered to the subject induces at least 80% CD28 receptor occupancy over the period of time between two administrations of said anti-CD28 Fab' antibody fragment. The higher doses administered according to the present invention (*i.e.,* 0.5 to 1.5 mg/kg per administration) could have been envisioned from the *in vitro* data that were available before the beginning of the phase I study disclosed below. However, to obtain 80% CD28 receptor occupancy, the skilled artisan would have administered 0.5 mg/kg at a frequency of once every two weeks (see Table 2 below), whereas the same dose can be administered once every 4 weeks or even less frequently according to the present invention. Still referring to Table 2 below, it appears that using doses of 1 mg/kg would have needed monthly administrations, whereas in the frame of the present invention, a dosage of 1 mg/kg every 8 weeks is sufficient. The same applies for doses of 1.5 mg/kg, for which the minimal frequency moves from once every 5 weeks to once every two months or more. Of course, the skilled artisan will adapt the frequency of administration to the doses which are administered, in order to obtain at least 80% CD28 receptor occupancy over the period of time between two administrations without administering an excessively high dosage. The higher the unitary dose administered, the lower the frequency of administration. Doses from 0.5 to 1 mg/kg will be administered, for example, at frequencies from once a month to once every two months, for example.

Examples of conditions susceptible of being improved or prevented by inhibiting a T cell immune response by a treatment with FR104 in accordance with the present invention are transplanted organ, tissue or cell dysfunction (including transplanted tissue rejection, in particular kidney transplant rejection, chronic allograft vasculopathy and graft-versus-host disease), T-lymphocyte-mediated autoimmune diseases, atherosclerosis, inflammatory diseases (including inflammatory bowel diseases such as ulcerative colitis and Crohn's disease) and type IV hypersensitivity. Among autoimmune diseases which can be treated according to the present invention, one can particularly cite autoimmune encephalomyelitis, rheumatoid arthritis, psoriasis, type 1 diabetes and multiple sclerosis. A further condition is psoriatic arthritis. Other autoimmune diseases can advantageously be treated according to the present invention; this is the case for bullous pemphigoid, acute disseminated encephalomyelitis (ADEM), ankylosing spondylitis, antiphospholipid antibody syndrome (APS), autoimmune oophoritis, celiac disease, gestational pemphigoid, Goodpasture's syndrome, Graves' disease, Guillain-Barré syndrome (GBS, also called acute inflammatory demyelinating polyneuropathy, acute idiopathic polyradiculoneuritis, acute idiopathic polyneuritis and Landry's ascending paralysis), Hashimoto's disease, idiopathic thrombocytopenic purpura, Kawasaki's disease, lupus erythematosus, myasthenia gravis, opsoclonus myoclonus syndrome (OMS), optic neuritis, Ord's thyroiditis, pemphigus, Reiter's syndrome, Sjögren's syndrome, Takayasu's arteritis, temporal arteritis (also known as "giant cell arteritis") and Wegener's granulomatosis. Autoimmune uveitis and alopecia areata are further autoimmune diseases which can be treated.

As already mentioned, the novel dosage regimen according to the invention is particularly advantageous for facilitating treatment administration. Monoclonal antibodies are usually administered intravenously over long treatment times of up to several years. Subcutaneous administration of drugs is an established therapy option for some chronic diseases such as diabetes mellitus, and it is an emerging route of administration for rheumatoid arthritis (Weinblatt et al., 2013) (Melichar et al., 2014). A major obstacle for subcutaneous approach in monoclonal antibody administration is the limitation of the volume which is administered. The upper limit of volume that can be painlessly injected is around 2 ml only. Higher injection volumes may cause pain and discomfort to the patient. In order to limit the volume of solution injected to the patient, highly concentrated solutions of monoclonal antibodies can be prepared. However, this imposes specific requirements on the drug formulation (Bowen et al., 2012).

Subcutaneous injection with therapeutic doses previously prescribed on the basis of the *in vitro* data was not possible. The therapeutic doses of FR104 according to the present invention can be included in a volume sufficiently small (about 2 ml) to consider this route of administration, for example for treating conditions such as psoriasis or rheumatoid arthritis.

According to a particular embodiment of the invention, the therapeutically effective amount of FR104 is administered to the subject in need thereof intravenously, subcutaneously, intramuscularly, topically or via intrathecal injection.

Diffusion of an antibody from the injection site may be an issue, especially when the antibody is administered via subcutaneous injection. A major advance has been associated with the introduction of hyaluronidase. Hyaluronan molecules represent an important component of the functional barrier preventing volume spread into the extracellular matrix. Because hyaluronan is constantly being renewed, a transient disruption does not pose a serious functional problem. Recombinant human hyaluronidase circumvents the problems of immunogenicity (Shpilberg and Jackisch, 2013). Recombinant human hyaluronidase (rHuPH20; Hylenex®) has been approved by the FDA as an adjuvant to facilitate subcutaneous administration of other agents. According to a particular embodiment the present invention, the composition administered to the human subject in need thereof comprises recombinant human hyaluronidase in addition to FR104.

The present invention also pertains to a pharmaceutical composition for inhibiting a T cell immune response in a human subject in need thereof, comprising FR104 in an amount comprised between 3 and 120 mg, for example between 3 and 100 mg, 3 and 80 mg, 3 and 60 mg or 3 and 40 mg, preferably less than 35 mg, such as less than 30 mg, less than 25 mg, less than 20 mg and for example between 3 and 14 mg, together with one or more pharmaceutically acceptable excipients. The pharmaceutical composition according to the invention is to be used as a unitary dose, which can be administered to a subject in need thereof at a frequency determined by a physician, but not higher than once a week. For example, the pharmaceutical composition according to the invention is a monthly unitary dose. Depending of the precise dosage and the clinical context (weight and general condition of the patient, nature of the disease, *etc.*)*,* the pharmaceutical composition according to the invention can be administered once a week or once every 2, 3, 4, 5 or 6 weeks. When the pharmaceutical composition according to the invention comprises 35 mg of FR104 or more, *i.e.,* between 35 mg and 120 mg of FR104, such as between 35 and 60 mg, 35 and 80 mg 35 and 100 mg, 40 and 120 mg, 60 and 120 mg, 80 and 120 mg, or 100 and 120 mg, it is preferably administered once every 3, 4, 5 or 6 weeks.

The pharmaceutical composition according to the present invention can be formulated so that it is suitable for intravenous, subcutaneous, intramuscular, topical or intrathecal administration. As mentioned above, the pharmaceutical composition according to the invention can comprise recombinant human hyaluronidase, especially when it is formulated for subcutaneous administration.

Another object of the present invention is a kit of parts comprising several doses of FR104, for example 2, 3, 4, 5, 6, 7, 8, 9, 10, 15 or more doses, wherein each dose comprises an amount of 3 to 120 mg of FR104 for example between 3 and 100 mg, 3 and 80 mg, 3 and 60 mg or 3 and 40 mg, preferably less than 35 mg, such as less than 30 mg, less than 25 mg, less than 20 mg and for example between 3 and 14 mg. These doses can be described, in the kit, as weekly doses, or doses to take up every 2, 3, 4, 5 or 6 weeks. According to a particular embodiment, they are monthly doses.

According to a particular embodiment of the pharmaceutical compositions and kits of parts described above, the pharmaceutical composition or each dose present in the kit are comprised in a syringe or any other device enabling its administration through the intravenous, subcutaneous, intramuscular or intrathecal routes.

Other characteristics of the invention will also become apparent in the course of the description which follows of the biological assays and first-in-human study, which have been conducted in the framework of the invention and which provide it with the required experimental support, without limiting its scope.

### EXAMPLES

### Example 1: FR104 pharmacokinetics, pharmacodynamics and product metabolism in humans

Before the beginning of the present study, no data were available on FR104 pharmacokinetics, pharmacodynamics and product metabolism in humans.

However, and in order to determine the Minimal Anticipated Biological Effect Level (MABEL), PK and PD data of cynomolgous and baboons studies were pooled together, analyzed by population PK/PD as well as allometrically scaled for humans. PK and RO data in Baboon and cynomolgous monkey were consistent and therefore modelled together.

### MABEL and estimation of the clinical dose levels

The Minimal Anticipated Biological Effect Level (MABEL) was chosen as the first dose level in the first-in-human trial of FR104 (EMA 2007: Guideline on strategies to identify and mitigate risks for first-in-humans clinical trials with investigational medicinal products, EMEA/CHMP/SWP/28367/07; FDA 2005: Guidance for Industry Estimating the Maximum Safe Starting Dose in Initial Clinical Trials for Therapeutics in Adult Healthy Volunteers).

The Minimal Anticipated Biological Effect Level (MABEL) is generally estimated as the dose generating approximately 10 to 20% of the maximal pharmacological activity. It is usually estimated based on *in vitro* data. *In vivo* data were used for FR104 given the very clear *in vivo* dose-response observed in baboons.

### Model development

In order to simulate CD28 receptor occupancy percentage over time for humans given different doses of FR104, all PK and PD data obtained so far in the cynomolgous and baboons studies (Baboon: 0.1, 1, 10 and 20 mg/kg single dose IV; Cynomolgus monkeys: 1, 2, 7 mg/kg single dose IV; Cynomolgus moneys: 20, 50, 100 mg/kg multiple dose IV) were analyzed using a population approach.

The following aspects were taken into account:
A mechanistic Pharmacokinetic/Target Engagement (PK/TE) model is used to predict efficacious dosing regimen in humans:
o Insufficient PK and RO data at low dose levels and low drug concentrations to allow reliable estimation of drug concentrations that result in 10% RO *in vivo*
o Sufficient PK and RO data for the higher drug concentrations to allow higher confidence modeling and simulation.

A formula to calculate maximal receptor occupancy based on the Kd of the mAb-target interaction was used to estimate MABEL (Duff, 2006). The following formula was used to calculate maximal receptor occupancy based on the Kd of the mAb-target interaction was used to estimate MABEL (Duff, 2006). $RO \left(%\right) = \frac{\left[Dose/V1\right]}{{K}_{D} + \left[Dose/V1\right]} \times 100$

The calculated RO at different dose levels are shown in the table below:

**Table 1: Estimated initial CD 28 RO%**

| Dose (µg/kg) | Initial RO (%) |
|---|---|
| 1.3 | 7 |
| 3 | 15 |
| 5 | 23 |
| 10 | 37 |
| 15 | 47 |
| 20 | 54 |
| 30 | 64 |
| 50 | 75 |
| 500 | 97 |

The conservative *in vitro* Kd value (4.6nM) and human plasma volume (V1, 0.04L/kg) were used. However, the *in vivo* Kd may be lower than the *in vitro* Kd. The targetmediated drug disposition (TMDD) model estimated V1=0.045 L/kg for FR104 (see below).

The following major model assumptions were used for this PK/TE TMDD analysis:
- Quasi-equilibrium approximation is suitable to describe the interaction between FR104 and CD28
- Treatment of FR104 does not alter the synthesis and degradation rate of CD28, or result in depletion of CD28-expressing cells, and binding of FR104 does not change the internalization rate of CD28 (kdeg = kint)
   o The maximum flow cytometry signal did not change after infusion of FR104
   o There was no drop in peripheral lymphocytes or CD4+ T cells after dosing of FR104
- The CD28 receptor levels were calculated based on the information provided and subsequently fixed (R0) in model fitting
- CD28 was assumed to express only in CD3+CD4+ T cells, while the expression on other cell types is not considered
- Target related parameters (Kd, Kdeg, R0) were assumed to be the same between baboon and cyno monkey.
- Individual body weight of animals is treated as a covariate for scaling of PK related parameters with an exponent of 0.75 (for CL and Q), and 1 (for V1 and V2).

The theoretical calculation of the CD28 receptor concentration in monkey when normalized to blood volume was based on the following data:
- In the studies reports, the receptor number per cell on CD3+CD4+ cells range from 20000 to 60000.
- In the model fitting, the receptor concentration was fixed at 0.4nM
- Other receptor concentrations (eg, 0.2nM) were fixed and it impacted Kdeg estimation inversely, while having less impact on model fitting at a certain range

The following formula was used and resulted in a correct fitting for receptor occupancy (Figure 2A): $Antigen conc . \left(nM\right) = \left(Receptor number per cell*blood cell density*{10}^{9}\right) / \left(6.023*{10}^{23}\right)$

### Projection of FR104 RO in Humans

The PK related parameters were scaled from monkey to human using allometric scaling (see below). The exponent was assumed to equal 1 for the volume of distribution. The exponent was assumed to equal 0.75 for systemic CL and distributional CL (Q). The average body weight of 70 kg for human, and 2.9 kg for cynomolgous monkey was used based on the mean body weight of monkeys in the single and multiple dose studies.
- Kdeg and Kd (which is affected by Kon and Koff) of CD28 were assumed to be the same between human and the animal species.
- The CD3+CD4+ T cell numbers (after body weight normalization) were comparable between monkeys and humans
- The CD28 receptor density was assumed to be 1.65 times higher compared to cyno/baboon.

A more detailed simulation of single doses show the following projected maximal receptor occupancy profiles in humans for different doses of FR104:

**Table 2: Simulation of CD28 occupancy % in humans after different single i.v. doses of FR104 (0.005 to 8 mg/kg) after different periods of observations (weeks)**

| Time (week) | 0.005 mg/kg | 0.05 mg/kg | 0.2 mg/kg | 0.5 mg/kg | 1 mg/kg | 2 mg/kg | 4 mg/kg | 8 mg/kg |
|---|---|---|---|---|---|---|---|---|
| 0 | 13 | 61 | 86 | 94 | 97 | 98 | 99 | 100 |
| 1 | 3 | 28 | 70 | 87 | 93 | 97 | 98 | 99 |
| 2 | 1 | 12 | 54 | 81 | 90 | 95 | 98 | 99 |
| 3 | 0 | 5 | 34 | 71 | 86 | 93 | 97 | 98 |
| 4 | 0 | 2 | 17 | 56 | 80 | 91 | 96 | 98 |
| 6 | 0 | 0 | 3 | 19 | 54 | 80 | 91 | 96 |
| 8 | 0 | 0 | 0 | 3 | 17 | 55 | 81 | 92 |
| 10 | 0 | 0 | 0 | 0 | 3 | 18 | 57 | 82 |
| 12 | 0 | 0 | 0 | 0 | 0 | 3 | 20 | 61 |

### Conclusion

Using the PK/TE modeling approach, it was determined that the selected MABEL dose of 0.005 mg/kg dose should lead to 13% of CD-28 receptor occupancy in humans as represented Figure 2A.

### Example 2: Study design

### 2.1. Principle

As described in Example 1, the dose selected as MABEL was of 5 µg/kg corresponding to approximately 13% of CD28 receptor occupancy.

It was decided to perform the dose escalating segment(s) of the study with single administrations. The duration of CD28 receptor occupancy on circulating blood T lymphocytes after FR104 administration is dose-dependent. In the toxicology study in cynomolgous monkeys, the infection/lymphomas mainly developed with 100% CD28 receptor occupancy on circulating blood T lymphocytes for at least 8/11 weeks, respectively.

In order to reduce the risks, the Applicant decided to first test very low doses inducing less than 4 weeks of 100% CD28 receptor occupancy (RO) on blood T lymphocytes and a maximum of approximately 50% RO at 8 weeks based on the PK/PD population modeling of the non -clinical data allometrically adapted for humans (see Table 2).

As a safety measure, the dose of 0.5 mg/kg was selected for holding the study and perform PK/PD and safety analyses before moving to the higher as well as repeat doses.

According to this simulation, the next doses of 1 and 2 mg/kg were to be adapted in order to comply with the rule of approximately 50% RO at 8 weeks post dose. An optional additional dose may have been tested. It would also comply with the rule of approximately 50% RO at 8 weeks postdose.

### 2.2 Materials and Methods

### Test Product, Dose, Mode of Administration

FR104 was administered intravenously at all dose levels by infusion of 100 mL in at least 30 min, after dilution to the right concentration in Ringer's lactate solution.

Six dose groups were scheduled for the Part 1, Cohort A (6 single ascending doses, only 4 of which were finally performed), with the following escalation scale based on a Minimum Anticipated Biological Effect Level (MABEL approach): 0.005; 0.050; 0.200; 0.500; 1.0; 2.0 mg/kg (the two last ones were cancelled after analysis of the PK/PD results obtained from groups 1-4).

Two dose groups for Part 1, Cohort B: 0.5 and 1.0 mg/kg were initially scheduled, and changed into 0.5 and 0.2 mg/kg, as explained below.

### Physical description of the study drug

GMP FR104 was provided to the site in 5 mL extractable volume vials containing 100 mg of FR104 (20 mg/mL) in 4 % mannitol + 0.02 % Tween 80. Appropriate Dilutions in Ringer's lactate solution was made on site.

All investigational products were prepared in accordance with Good Manufacturing Practice (GMP) as required by the current GCP.

### Other medication administered in the study

The matching placebo injection contains vehicle (Ringer's lactate solution). For placebo dosing, 100 mL vehicle was administered.

### Pharmacokinetic calculations

Pharmacokinetic calculations were performed using Phoenix WinNonlin 6.2 or higher (Pharsight Corporation, Palo Alto, CA, USA).

The following individual PK parameters, where appropriate, were determined for FR104 from individual concentration-time profiles in serum, using a non-compartmental method:
Cₘₐₓ: the maximum observed serum concentration
tmax the time of occurrence of Cₘₐₓ

AUC_{inf} the area under the serum concentration vs time curve from time zero to infinity, calculated from AUC₀₋ₜ + (Cₜ/λ_{z}), where Cₜ is the last observed quantifiable concentration and λ_{z} the first order terminal rate constant

AUC_{0-28d} area under the serum drug concentration-time curve over the dosing interval (*i.e.,* 28 days) calculated by the linear-logarithmic trapezoidal rule
t_{1/2} terminal half-life, calculated from (In 2)/λ_{z}
R_{ac} accumulation ratio, calculated as AUC_{0-28d} Day 29/AUC_{0-28d} Day 1 (Part 2 only)
CL systemic clearance calculated as Dose i.v./ AUC_{inf}
V_{z} volume of distribution calculated as CL/λ_{z}
Dose normalized parameters (Cₘₐₓ/dose, C_{avg}/dose, AUC_{0-28d} /dose) were assessed.

Other PK parameters were calculated as deemed appropriate.

### Pharmacodynamics

### CD28 Receptor Occupancy

The primary PD variable is cluster of differentiation (CD)28 RO over time, evaluated as RO by FR104 in blood samples collected at each time point during the study using a fit-for-purpose validated method. The appropriate PD parameters was calculated.

### Keyhole Limpet Hemocyanin Challenge

For Part 1, Cohort B only.

Human anti-KLH Abs in serum were detected using the Enzyme-Linked Immuno Sorbent Assay (ELISA) method.

Blood samples of 1.5 mL were collected by venipuncture or via indwelling cannula in the forearm into standard serum tubes.

### Anti-KLH antibody measurements by ELISA

Analysis of the serum study samples was performed by using commercial kit "Human anti-KLH IgG" catalogue N° 700-140-KLG from Alpha Diagnostic International. The lower and upper limits of quantification were determined to be 7.20 and 86.0 U/ml. This Elisa kit is based on the binding of human anti-KLH antibodies in samples to KLH antigen immobilized on the microwells, and anti-KLH IgG antibody is detected by anti-human IgG-specific antibody conjugated to HRP enzyme. After a washing step, chromogenic substrate (TMB) is added to terminate the reaction and absorbance at 450 nm is then measured using an ELISA microwell reader.

### Blood/Serum tests

Whole blood samples were obtained from different donors and collected on K2-EDTA or K3-EDTA blood collection vacutainer tubes. Samples for the determination of FR104 PK and CD28 receptor occupancy were obtained from blood collected at the following time points:
Day 1, predose and at 0.5, 0.75, 1, 2, 4 et 8 hours after start of infusion
Day2, 24 hours after start of infusion
Day 3, 48 hours after start of infusion
Day 5, 96 hours after start of infusion
Day 8, 1 week after start of infusion
Day 15, 2 weeks after start of infusion
Day 29, 3 weeks after start of infusion
Day 43, 6 weeks after start of infusion only for 0.5 mg/kg (Group 4 cohort A)
Day 57, 8 weeks after start of infusion, not to be performed if dose < 0.2mg/kg
Day 85, 12 weeks after start of infusion, not to be performed if dose < 0.2mg/kg
Day 113, 16 weeks after start of infusion (Follow up visit for group with a dose ≥ 1. mg/kg)

### FR104-PEG concentration measurement by ELISA

The FR104 (anti-CD28.3 Fab) molecule was incubated in CD28Fc-coated plates. Bounded FR104 was then detected with a rabbit monoclonal antibody directed to PEG (methoxy group), which was revealed by an anti-rabbit polyclonal antibody labeled with peroxidase.

### Reagents and buffers

- CD28- Ig (CD28/Fc Chimera, R&D Systems # 342-CD-200, reconstituted at 200µg/mL in PBS)
- Coating buffer: NaHCO₃ 0.05 M pH 9.2 (80mL Na₂CO₃ 0.05M + 920ml NaHCO₃ 0.05M, pH9.2)
- BSA (Sigma # A-7906)
- Tween 20 (Sigma # P7949)
- Sample
- Standard: FR104 #CAA-1 (0.5mg/ml)
- Monoclonal antibody Rabbit anti-PEG (methoxy group) (Epitomics #2061-1, 0.97mg/ml)
- Polyclonal goat anti-rabbit labelled peroxidase (Jackson Immunoresearch #111-035-144, 0.8 mg/ml)
- Substrate: TMB (Sigma # T8665)
- H₂SO₄ (VWR 20704.292)

Plates were coated with CD28-Ig at 1 µg/ml in carbonate buffer 0.05M pH 9.2 (50 µL/well) and incubated 2h at 37°C or overnight at 4°C. Wells were emptied and washed 3 times successively with 200 µL PBS-0.05% Tween. 100µL of PBS Tween 0.1% BSA 1% were added and plates were incubated for 1h at 37°C.
- *Standard:* FR104 #L27221/G1/2 (10.01mg/ml) was diluted for the first point at 100 µg/ml and serially diluted by a factor of 5 for the 7 following points, in duplicate. The serum (50 µl) was diluted at least at 1/10 in PBS-0.1% Tween and incubated 1h at 37°C. Wells were emptied and washed 3 times successively with 200 µL PBS-0.05% Tween and diluted. 50µl /well of monoclonal antibody Rabbit anti-PEG at 1/500 in PBS-0.1%Tween was then added, followed by incubation for 1h at 4°C. Wells were emptied and washed 3 times successively with 200 µL PBS-0.05% Tween. A goat anti-rabbit peroxidase (anti-Rb PO) at 1/2000 in PBS-0.1% Tween (50µL/puits) was added and incubated 1h at 4°C. (washing step) TMB (50 µL/well) was added and incubated 10 min at room temperature in the dark. The Stop buffer was added (50 µL/well H₂SO₄ 0.5 M). Measurement of the absorbance at 450 nm, (ref 630nm) was then acquired using a Microtiter plate (*Nunc Immunoplate,* Nunc, # 442404). The SoftMaxPro programme was used for analysis and to determine FR104 concentrations in the samples.

### Receptor expression measurement by Flow Cytometry

### Receptor occupancy determination:

Each blood sample was split in half and mixed or not with the FR104 antibody. Upon red blood cell lysis, FR104 binding to CD28 was investigated by Flow cytometry on lymphocytes stained with a FITC conjugated anti-CD3 antibody, using successively an anti-PEG rabbit monoclonal antibody and a Alexa fluor 405-conjugated anti rabbit secondary antibody. Thus, the mean fluorescent intensity (MFI) of the PEG staining was determined in both wells containing the second half of blood volume, from the same donor supplemented with an excess of FR104 antibody. FR104-promoted CD28 saturation was then calculated for each blood donor by performing the ratio between the MFI of both wells (without FR104 excess/with excess of FR104).

### Working solution and buffers

**Table 3**

| **Reagent** | **Supplier** | **Reference** | **Storage Condition** |
|---|---|---|---|
| NH₄Cl | Sigma | A9434 | RT |
| KHCO₃ | Sigma | 60339 | RT |
| Na₂-EDTA | Sigma | E1644 | RT |
| Sodium Azide | Sigma | S2002 | RT |
| HCl | Merck | 1.09063.1000 | RT |
| PBS (w/o Mg²⁺, Ca²⁺) IX | Gibco | 14190-094 | RT before opening and at RF upon opening |
| BSA-7.5% | Sigma | A8412 | RF |
| Alexa Fluor 405 goat anti-rabbit IgG | Molecular probes | A31556 | RF |
| FITC mouse anti-human CD3 IgG | BD Biosciences | 556611 | RF |
| Rabbit anti-PEG monoclonal IgG | Abcam | 51257 | FZ |
| BD C&ST beads | BD Biosciences | 650621 | RF |
| FACS Rinse solution | BD Biosciences | 340346 | RT |
| FACS Clean solution | BD Biosciences | 340345 | RT |
| FACS Flow Sheath fluid | BD Biosciences | 342003 | RT |

| | | | |
|---|---|---|---|
| RF: Refrigerator (+5°C±5°C), RT: Room Temperature; FZ: (-24°C±6°C) | | | |

### Preparation of reagents and solution

**Table 4**

| **Solutions and buffers** | **Components** | **Preparation** | **Storage conditions** |
|---|---|---|---|
| 1X Red Blood Cells Lysis solution | NH₄Cl, KHCO₃, Na₂-EDTA and dd-H₂O | 1.658 g NH₄Cl, 0.2 g KHCO₃, 0.00744 g Na₂-EDTA, 200 mL dd-H₂O. Adjust pH between 7.2 and 7.4 | Up to 4 weeks, +5°C±5°C |
| FACS Buffer | DPBS (w/o Mg²⁺, Ca²⁺) 1X, Bovine Serum Albumin, Sodium Azide | 40 mL of 7.5% BSA solution, 0.3 g Sodium Azide, s.q.f 300 mL DPBS IX | Up to 4 weeks, +5°C±5°C |

### Preparation of diluted antibody solution

| **Antibody diluted solution** | **Components** | **Preparation** | **Storage Conditions** |
|---|---|---|---|
| Rabbit anti-PEG IgG diluted solution | Rabbit anti-PEG IgG stock solution, FACS Buffer | 5 µL Rabbit anti-PEG IgG, 254 µL FACS Buffer | No storage, freshly prepared |

### Preparation of antibody working solutions

| **Antibody working solution** | **Components** | **Preparation** | **Storage Conditions** |
|---|---|---|---|
| FITC mouse anti-human CD3 IgG/Rabbit anti-PEG IgG working solution | Rabbit anti-PEG IgG diluted solution, Mouse anti-human CD3 FITC labelled IgG stock solution, FACS Buffer | 90 µL Rabbit anti-PEG IgG, 240 µL Mouse anti-human CD3 FITC labelled IgG, 270 µL FACS Buffer | No storage, freshly prepared |
| Rabbit anti-PEG IgG working solution | Rabbit anti-PEG IgG diluted solution, FACS Buffer | 90 µL Rabbit anti-PEG IgG, 510 µL FACS Buffer | No storage, freshly prepared |
| Alexa Fluor 405 Goat anti-rabbit IgG working solution | Alexafluor 405 Goat anti rabbit IgG stock solution, FACS Buffer | 3 µL Goat anti-rabbit IgG stock solution, 597 µL FACS Buffer | No storage, freshly prepared |

### FR104 dilution table

**Tables 5-7**

| **Tube** | **Concentration (µg/mL)** | **From** | **From concentration (µg/mL)** | **µL** | **Volume of DPBS (µL)** | **Final Volume (µL)** | **Final concentration in assay with cells (µg/mL)** |
|---|---|---|---|---|---|---|---|
| S2 | 500 | S1 | 21000 | 3 | 123 | 126 | - |
| CO1 | 50 | S2 | 500 | 20 | 180 | 200 | 5 |

| | | | | | | | |
|---|---|---|---|---|---|---|---|
| Table given for a FR104 stock solution (S1) at 21 mg/mL, and may be then adjusted depending on the FR104 batch stock concentration. CO: concentration | | | | | | | |

The assay was performed in triplicate for test samples treated or not with the FR104 saturating dose (5µg/ml). Samples were dispensed onto-96 well plates.

The appropriate volume of an FR104 solution at 50 µg/ml was performed and gently homogenized in EDT-treated blood tube. 50 µl of each human whole blood sample was plated into 6 wells of a 96 V-bottom well plate. 5.5µl of FR104 (50µg/ml in D-PBS) was added in first set of three well and 5.5µl of D-PBS in the second set of three wells. After gently homogenizing, plates were incubated 15 minutes at room temperature (RT). 150µl/well of red blood cells lysis solution was added and then centrifugated 1min at 1200g. Supernatant was discarded and 150µl/well of red blood cells lysis solution was added. After 5minutes at RT, plates were centrifuged 1minute at 1200g (repeat 3 time). Then 150µl of FACS buffer was added and centrifuged. 30µl/well of the mix solution of FITC mouse anti-human CD3 IgG/Rabbit anti-PEG IgG working solution was added and incubated 15 minutes in the dark at 4°C. Cells were washed with FACS buffer and centrifuged, step repeated twice. 30µl/well of Alexa Fluor 405 Goat anti-rabbit IgG working solution was added and incubated 15 minutes at 4°C in the dark. Cells were washed twice with FACS buffer. Cells were then resuspended into 300µl of FACS buffer for FACS analysis.

### 2.3 Study Design

This FR104 phase I study design is cautious and in line with the European Guideline for First-in-Human clinical trials with investigational medicinal products (EMEA/CHMP/ SWP/28367/07).

This study is a first-in-human, phase I, randomized, double-blind, placebo-controlled, single center study evaluating single and multiple ascending intravenous doses of FR104 in healthy subjects.

Up to 71 healthy male and female subjects were selected according to the inclusion and exclusion criteria, *i.e.,* 57 subjects in Part 1 (SAD: 3 cohorts of 36 [Cohort A], 14, [Cohort B], and 7 subjects [optional Cohort C], respectively). The anticipated total duration of the study was 9 months (36 weeks).

All FR104 doses were administered intravenously by a slow infusion of at least 30 minutes. A staggered approach was observed within all dose levels. An interval of at least 14 days (last to first administration) was observed between all dose levels. Individual subjects on a same day of dosing were dosed at least 60 minutes apart.

### Dose Selection

The first dose of the planned first-in-human study was based on the MABEL as determined by the PK/PD population modelling of the nonclinical data allometrically adapted to humans (see Example 1 above for details). The dose selected was of 5 µg/kg corresponding to an estimated approximately 13 % of CD28 receptor occupancy (RO).

The dose escalating segment(s) of the study were performed with single administrations. In order to reduce the risks, a hold of approximately 12 weeks was scheduled after the 1 mg/kg dose level, *i.e.,* before the initiation of dose levels supposed to induce 100% CD28 RO over a period of time of 4 weeks or more (according to the population PK-PD modeling). This period of 4 weeks was considered short enough to make it very unlikely in man that a viral reactivation generates any pathology. The 12 weeks hold enabled PK/PD and safety evaluations before moving to the higher doses of 1, 2 mg/kg which were initially scheduled.

According to the simulation, doses of 1 and 2 mg/kg were supposed to induce, respectively, 84 and 92% of RO at 4 weeks and 18 and approximately 50% RO at 8 weeks. As a safety measure the dose of 0.5 mg/kg has been selected for holding the study and performing PK/PD and safety analyses before moving to the higher doses.

### Part 1 Single Ascending Dose (SAD) part

Subjects were recruited on the basis of their medical history and health status as judged by the Principal Investigator (or designated co-investigator). During each treatment period, subjects were housed at the study center from the day before dosing (Day-1) until Day 5. The meals were standardized during the residential period. For Groups 1 - 4 (0.005; 0.05; 0.2 and 0.5 mg/kg), ambulatory visits were planned at Day 8, Day 15, Day 29, and Day 57. For Group 4 (0.5 mg/kg), an extra ambulatory visit was planned at Day 43. The last ambulatory visit was followed by a follow-up visit at Day 85 (Week 12). Groups 5 and 6 should have been dosed with 1 and a maximum of 2 mg/kg FR104, respectively, but the results obtained with lower doses led the investigators to cancel these doses.

### Cohort A

Twenty two subjects were enrolled in one of the 4 dose level groups in Cohort A. Sequential (dose level) groups of healthy subjects received increasing doses of FR104 administered IV.

In the first 2 dose level groups (Group 1 to 2), 4 subjects, each, were randomized to either FR104 or placebo in a 3:1 ratio so that 3 subjects received FR104 and 1 subject received placebo.

A staggered dose approach was applied, *i.e.,* one subject was dosed followed by a second subject 48h later, and the two remaining subjects of the dose level group 48h after the second subject (total 5 days):
Day 1: n=1
Day 3: n=1
Day 5: n=2

In Groups 3 and 4, 7 subjects, each, were randomized to either FR104 or placebo in a 5:2 ratio so that 5 subjects received FR104 and 2 subjects received placebo. The randomization ensured that in the first group of 2 subjects 1 received FR104 and 1 received placebo.

A staggered dose approach was applied, *i.e.*, 2 subjects were dosed followed by 2 subjects 48h later, and the remaining 3 subjects of the dose level group 48h after the fourth subject (total 5 days):
Day 1: n=2
Day 3: n=2
Day 5: n=3.

The same protocol was scheduled for groups 5 and 6 (7 subjects, each, with a dose level of 1 mg/kg and 2 mg/kg) but was cancelled after analysis of the data for the first 4 groups.

**Table 8: Single ascending dose**

| **Cohort A** | **Dose (mg/kg)** | **FR104 number of subjects** | **PLACEBO number of subjects** |
|---|---|---|---|
| Group 1 | 0.005 | 3 | 1 |
| Group 2 | 0.050 | 3 | 1 |
| Group 3 | 0.200 | 5 | 2 |
| Group 4 | 0.500 | 5 | 2 |
| Approximately 12 weeks hold for PK, PD and safety evaluation | | | |

### Cohort B

Fourteen subjects were enrolled in one of the 2 dose level groups (7 in each group) in Cohort B.

The subjects were randomized to either FR104 or placebo in a 5:2 ratio so that 5 subjects received FR104 and 2 subjects received placebo. The randomization ensured that in the first group of 2 subjects, 1 received FR104 and 1 received placebo.

In addition to the common assessments, subjects in this cohort received a keyhole limpet hemocyanin (KLH) challenge on the day of FR104 injection.

A staggered dose approach was applied, *i*.*e*., two subjects were dosed followed by two subjects 48h later, and the three remaining subjects of the dose level group 48h after the fourth subject (total 5 days):
Day 1: n=2
Day 3: n=2
Day 5: n=3

The initial design was as follows:
KLH challenge, single dose.

**Table 9: initial design for SAD including KLH challenge**

| Part 1 | | Single Ascending Dose | |
|---|---|---|---|
| Cohort B | Dose (mg/kg) | FR104 number of subjects | PLACEBO number of subjects |
| Group 7* | 0.500 | 5 | 2 |
| Group 8* | 1.000 | 5 | 2 |
| *°healthy subjects naive to KLH will receive a KLH challenge | | | |

This design was modified to take the results obtained in Groups 1-4 into account:
KLH challenge, single dose.

**Table 10: modified design for SAD including KLH challenge**

| **Cohort B** | **Dose (mg/kg)** | **FR104 number of subjects** | **PLACEBO number of subjects** |
|---|---|---|---|
| Group 7* | 0.500 | 5 | 2 |
| Group 8* | 0.200 | 5 | 2 |
| Group 9* | 1,5 | 5 | 2 |
| Group 9 bis* | 0.02 | 5 | 2 |
| *healthy subjects naïve to KLH received a KLH challenge | | | |

### Inclusion Criteria

Subjects meeting all of the following criteria are eligible to participate in this study:
1. Male or female, aged 18 to 60, extremes included;
2. In good health condition [medically stable] as determined on the basis of medical history, vital signs, clinical laboratory testing, and general physical examination performed at screening;
   Note: a retest can be done in case of an out of range clinical laboratory test value that will determine a subject's eligibility. This retest should preferably be done at an unscheduled visit. The result of the retest will be considered for subject eligibility. If the retest is outside normal reference ranges, the subject may be included only if the investigator judges the abnormalities to be not clinically significant.
3. Electrocardiogram (ECG) within normal range, or showing no clinically relevant deviations, as judged by the investigator;
   Note: a retest can be done in case of an out of range ECG value that will determine a subject's eligibility.
4. Weighs at least 50 kg and no more than 100 kg and has a Body Mass Index (BMI) within normal range: 18.0≤BMI<30.0 kg/m2;
5. Negative urine test for selected drugs of abuse at screening;
6. Negative alcohol breath test at screening;
7. Female subject is postmenopausal or surgically sterile (having had a hysterectomy, bilateral oophorectomy, or tubal ligation);
8. Female subject has a negative pregnancy test at screening;
9. Non-vasectomized male subjects having a female partner of childbearing potential must agree to the use of an effective method of contraception until 90 days after the last administration of study drug;
10. Male subject has to agree not to donate sperm until 90 days after the last administration of study drug;
11. Willing to adhere to the prohibitions and restrictions specified in this protocol;
12. Informed Consent Form (ICF) signed voluntarily before any study-related procedure is performed, indicating that the subject understands the purpose of and procedures required for the study and is willing to participate in the study;
13. Subjects should be EBV-positive as per PCR;
14. Nonsmoker or light smoker, *i.e.,* smokes maximal 5 cigarettes (or 3 cigars or 3 pipe-full) per day, and ability and willingness to refrain from smoking during confinement and ambulant visits in the clinical research center.
   For Part 1, Cohort B only:
15. The subject did not undergo a KLH challenge.

### Example 3: Initial Results of the Single Ascending Dose (SAD) part of the study

The concentration of FR104 was measured in the blood of patients by ELISA and the level of Receptor Occupancy was measured by Flow Cytometry on days 0, 1, 2, 5, 8, 15 and 29 after single ascending doses. Pharmacokinetic results are represented in Figure1 in ng/ml of FR104 in the plasma and pharmacodynamics results as percentage of the occupancy of CD28 receptors on target CD3+ T cells. Figure 1A shows FR104 concentration after single i.v. FR104 injections of 0.005, 0.05, 0.2 and 0.5 mg/Kg, administered over 30 minutes, peak plasma concentrations measured 2 hours post-injections reached 117 ± 11, 1012 ± 48, 4922 ± 853 and 11620 ± 1076 ng/ml, respectively. From plasma concentrations measured over the first month, half-life was shown to be dose-dependent with values of 3.4 days for the 0.05 mg/Kg dose, 5.7 days for the 0.2 mg/Kg dose and 7.9 days for the 0.5 mg/Kg dose. Figure 1.B surprisingly shows that the Receptor Occupancy reached 80% after a single i.v. injection of 0.005 mg/Kg of FR104 and 100% after a single i.v. injection of 0.05 or 0.2 mg/Kg of FR104 rather than about 13% of RO for 0.005 mg/kg of FR104 as expected. The CD28 receptor became fully desaturated after 15 days post- i.v. injection of 0.005 mg/Kg and 28, 56 and 84 days posti.v. injections of 0.05, 0.2 and 0.5 mg/Kg.

Figure 2 shows the different RO percentages at different FR104 concentrations previously determined with *in vitro* results (figure 2A) compared to RO percentage observed after clinical trial. Figure 2B compares the predicted RO % determined with the modified FR104's Kd following the analysis of the first cohort A of the clinical study with the observed RO% calculated by Flow cytometry as explained in the material and method. This figure shows the perfect correlation between both situations, confirming that the Kd measured *in vitro* in Poirier et al, 2012 was underestimated for Human use and that the FR104 dissociation constant is about 0.3 nM rather than 4,6 nM (10 times lower). These results were not predictable because the RO % determined in the non-human primate model fitted very well with predictions. The FR104 therapeutical doses calculated in 2012 for preclinical studies showed good efficacy and were abundantly published ((Haanstra et al, JI 2015) (Poirier et al, JI 2015) (Poirier et al, Experimental Dermatol. 2015) (Vierboom et al, Clinical and experimental immunol 2015)).

The expression of CD28 at T cell surface was then analysed during 1 month after FR104 infusion by flow cytometry. Figure 3 shows the Mean of Fluorescence of the pegylated FR104 reflecting the presence of the CD28 receptor at cell surface. The results show that in the 4 different groups of volunteers receiving FR104, CD28 is still present at T cell surface even after FR104 binding, underlying that FR104 does not induce receptor internalization nor down-regulation.

KLH challenge was made on a FR104 single dose infusion (0.02, 0.2, 0.5 and 1.5 mg/kg modified concentrations) to test the immune response to KLH antigen in human. The anti-KLH antibody concentration was analysed by ELISA using a commercial kit. Figure 4 represents the anti-KLH antibody response. This result indicates a FR104 dose dependent inhibition of anti-KLH response compared to volunteers receiving placebo. From 0.2mg/kg therapeutical dose, the response was down regulated. 100% of inhibition was observed for volunteers receiving 0.5mg/kg of FR104 . The new therapeutical FR104 doses are effective from 0.02mg/kg to inhibit immune system.

### Example 4: First-in-Man Study report

### METHOD

### Study design

This study is a first-in-human, phase I, randomized, double-blind, placebo-controlled, single centre study evaluating single and multiple ascending intravenous doses of FR104 in healthy subjects. It was approved by the Ziekenhuisnetwerk Antwerpen Independent Ethics Committee (Protocol Number FR104-CT01; EudraCT Number 2015-000302-19; Clinical-Trials.gov identifier: NCT02800811). The study was conducted at SGS Life Science Services, Clinical Pharmacology Unit Antwerp -Antwerp, Belgium, in compliance with the Good Clinical Practice guidelines and the principles of the Declaration of Helsinki.

### Participants

Eligible male and female subjects gave written informed consent and were in good health. Key inclusion criteria were general good health, 18-60 years of age, ≥50 kg and no more than 100 kg with a BMI of 18-30 kg/m². Exclusion criteria were any significant past medical history or abnormal laboratory tests.

### Intervention

The test drug was FR104 (supplied by OSE Immunotherapeutics SA., Nantes, France), while the comparator drug (matching placebo) was Ringer's lactate solution. FR104 was supplied as 5-mL solution containing FR104 100 mg (20 mg/mL) and stored at 2-8°C (36-46_{°}F) protected from light. Syringes with the appropriate dilution or placebo were prepared by SGS pharmacy and provided to the investigator in a double-blind manner.

The doses were initially selected based on population PK-PD modeling of non-human primates data (with allometric adaptation) and human data were later included after the interim analysis. The initial dose (MABEL, 0.005 mg/kg) corresponded to a modeled CD28 RO at Cmax of approximately 20%.

For dose levels ≤0.05 mg/kg, FR104 was administered by IV infusion of 10 mL in at least 30 min, after dilution to the correct concentration in Ringer's lactate solution. For other dose levels, FR104 was administered by IV infusion of 100 mL in at least 30 min, after dilution to the correct concentration in Ringer's lactate solution.

### Treatment regimens

50 subjects were to be selected in Part 1 (SAD: 2 cohorts of 22 [Cohort A] and 28 [Cohort B] subjects) and 14 subjects in Part 2 (MAD). During each treatment period, subjects were housed at the study center from the day before dosing (Day-1) until Day 5. A staggered dose approach was applied, ie, 1 or 2 subjects were dosed (Day 1) followed by 1 or 2 subjects 48 h later (Day 3), and the remaining subjects of the dose level group 48 h after the fourth subject. The total duration of the study was 36 weeks, including 11 week follow-up after the last repeat dose. An interval of at least 14 days (last to first administration) was applied between all dose levels. Individual subjects on a same day of dosing were dosed at least 60 minutes apart.

***Part 1: Single Ascending Dose Part (SAD)* -** Twenty-two subjects were to be enrolled in one of the 4 dose level groups in Cohort A: 0.005 mg/kg in Group 1, 0.050 mg/kg in Group 2, 0.200 mg/kg in Group 3, and 0.500 mg/kg in Group 4. In Groups 1 and 2, 4 subjects each were randomized to either FR104 or placebo in a 3:1 ratio so that 3 subjects received FR104 and 1 subject received placebo. In Group 3 to 4, 7 subjects each were randomized to either FR104 or placebo in a 5:2 ratio so that 5 subjects received FR104 and 2 subjects received placebo. The randomization ensured that in the first group of 2 subjects, 1 received FR104 and 1 received placebo. A predefined hold of approximately 12 weeks was made after dosing in group 4, for interim evaluation PK, PD and safety based on which decisions for the next dose levels were made. Twenty-eight subjects were to be enrolled in one of the 4 dose level groups (7 in each group) in Cohort B: 0.500 mg/kg in Group 7, 0.200 mg/kg in Group 8, 1.5 mg/kg in Group 9, and 0.020 mg/kg in Group 9 bis. The subjects were randomized to either FR104 or placebo in a 5:2 ratio. In addition to the common assessments, subjects in this cohort received a KLH challenge on the day of FR104 injection.

***Part 2: Multiple Ascending Dose Part (MAD)-*** Fourteen subjects were to be enrolled in one of the 2 dose level groups (7 in each group) in Part 2: 0.2 mg/kg in Group 10 and 0.5 mg/kg Group 11. Each subject received 2 administrations of FR104 or placebo separated by an interval of 28 days.

### Cytokines assessment

Blood samples were collected predose and postdose at hours 1, 2, 4, 8, 24 (day 2). Samples were also collected on day 15, 43, 57, 85, 113. Cytokines IFN-γ, IL-1β, IL-2, IL-4, IL-6, IL-8, IL-10,

IL 12p70 AND TNF-α were dosed in serum using a validated ECLIA method using the commercial kit Pro Inflammatory Panel I V-plex assay (# K15049G from Meso Scale Discovery, Rockville, MA). MULTI-SPOT® plates are pre-coated with capture antibodies. Samples are loaded onto wells with a solution containing detection antibodies conjugated with electro-chemiluminescent labels (MSD SULFO-TAG™). Analytes in the sample bind to capture antibodies immobilized on the working electrode surface; recruitment of the detection antibodies by the bound analyte complete the sandwich. After addition of MSD Reading Buffer a voltage is applied to the plate electrodes and light emission occur if significant SULFO-TAG™ labelled antibodies has bound to the plate. The instrument measures the intensity of emitted light to provide a quantitative measure of the analyte in the sample.

### PK analysis

Serial blood samples for PK assessments were collected at the following timepoints relative to infusion start time: predose and postdose at hours 0.5, 0.75, 1, 2, 4, 8, 24 (day 2), 48 (day 3), days 5, 8, 15, 29, 43, 57, 85, 113 (>0.5 mg/kg dose groups only). Concentrations of FR104 in serum were determined using a validated method involving quantitative Electrochemiluminescence Immunosorbent Assay (ECLIA) on a MSD Sector Imager 6000 (MSD, Gaithersburg, MD, USA). Pharmacokinetic calculations were performed by SGS-LSS using Phoenix WinNonlin 6.2 or higher (Pharsight Corporation, Palo Alto, CA, USA). The lower and upper limits of quantification (LLOQ and ULOQ) for the assay were 100 and 2000 ng/mL, respectively. The overall precision and accuracy of the quality controls and standards were ≤20% and within ±20%, respectively. A standard curve was constructed enabling sample concentrations to be estimated by interpolation from the fitted curve.

### PD analysis

Blood samples were collected at the same timepoints as for PK analyses for CD28 receptor occupancy (RO), using a partially validated method involving cytofluorymetry. Each blood sample to be tested was split in half and one sample was mixed with an excess of FR104. Upon red blood cell lysis, FR104 binding to CD28 was investigated by flow cytometry, on lymphocytes stained with a FITC conjugated anti-CD3 antibody, using successively an anti-PEG rabbit monoclonal antibody and an Alexa fluor 405-conjugated anti rabbit secondary antibody. Thus, RO was calculated by performing the ratio between the MFI of the well without excess of FR104 and the well with an excess of FR104. The sensitivity of the method was 0.25 µg/mL of FR104. Intra-donor replicates precision ranged between 1% to 9% (CV) and interdonor precision ranged between 16% and 22% (CV). CD28 expression level was measured by the MFI of FR104 in excess conditions.

Peripheral T lymphocyte sub-populations and activation status were assessed by flow cytometry on stabilized whole blood using TransFix/EDTA vacuum blood collection tubes (Cytomark, Buckingham, UK). CD45, CD3, CD28, CD45RO, CD4, CD8, CD25, CD127, CD69 and CCR7 markers were measured to define the following subpopulations: naïve T cells, activated T cells, memory T cells, central memory T cells, effector memory T cells and TEMRA, in the CD4+ and CD8+ compartments. nTreg have also been recorded.

All other laboratory testing including EBV PCR and EBV IgG and IgM antibodies have been performed by the clinical biology laboratory of the investigational Center, using standard procedures.

Immune responsiveness of blood cells was investigated ex-vivo. Blood samples were collected by venipuncture or via indwelling cannula in the forearm into TruCulture® blood collection tubes (Myriad RBM, Austin, Tx) containing SEB + LPS stimuli and into control tubes, for each blood draw. Tubes were maintained at 37°C for 24h before plasma was mechanically extracted and frozen until analysis of IL-2, IFNg and IL-8 cytokines. Cytokines were analyzed using the same ELISA method used to assess cytokines in the serum.

### Immunoassessment

Blood samples for anti-FR104 antibody detection were collected at screening and on day 1 (predose), days 15, 29, 57, 85 and/or 113. Tittering of anti-FR104 antibodies in serum was performed using a validated electrochemiluminescence, bridging immunogenicity assay. The method used an acidic treatment of the serum samples to allow when necessary dissociation of FR104/FR104-ADA followed by a single step assay bridging format whereby anti-FR104 antibodies (ADA) are captured in solution by a combination of biotinylated and sulfo-TAG labelled forms of FR104. Complex formation is subsequently detected by ECL onto the MSD platform. The sensitivity of the assay was 3.46 ng/ml (with a CV of 31%) and the mean drug tolerance was 179 µg/ml (with a CV of 26%).

### KLH immunisation

Blood samples for anti-KLH antibody detection were collected at screening and on days 15, 29, 57, 85 and/or 113. Anti-KLH antibodies were dosed by a qualified ELISA in serum using commercial kit "Human Anti-KLH IgG" (# 700-140-KLG from Alpha Diagnostic International, San Antonio, Tx).

### Statistical methods

The sample size was determined for this study based on a precedent set by other Phase 1 studies similar in design and after consultation of the Medicine and Health Care Regulatory Agency (UK), the Paul Ehrlich Institute (Germany), the Federal Agency for Medicines and Health Products (Belgium) and the European Medicines Agency (EMA). A sample size of 64 healthy volunteers was deemed sufficient to meet the objectives of the protocol. All statistical calculations were performed using the SAS (version 9.2) software for statistical computations, and SAS for graphical purposes.

### RESULTS

### Participant flow

The study was conducted at 1 clinical center from 27 March 2015 to 19 February 2016. A total number of 65 subjects was divided over 2 study parts. In Part 1, 37 subjects were administered a single IV dose of FR104 (ranging from 0.005 to 1.5 mg/kg) and 14 subjects were administered a single IV dose of placebo. In Part 2, 10 subjects were administered two doses of FR104 (ranging from 0.200 to 0.500 mg/kg) and 4 subjects were administered two IV doses of placebo, separated by an interval of 28 days. All but 1 subject (in Part 1, Cohort A, Group 2) received study drug as planned (see above). All subjects were randomized and treated. All subjects completed the study and were included for the safety analysis. One subject (Part 1, Cohort A, Group 2) was excluded from the PK and PD analyses due to a protocol deviation related to a dysfunction of the infusion pump. Due to this incorrect dose administration, one additional subject was included in Group 2. One subject in Part 2 did not receive the second dose of FR104 0.500 mg/kg due to receiving prohibited concomitant medications following a treatment unrelated AE. The subject completed all visits as per protocol. The subject was not excluded from the PK and PD population, but the data taken after Day 29 were excluded from the analysis. There were no subjects for whom the blinding code was broken by the Investigator or the Sponsor.

### Baseline data

Subject demographics are shown in Table 11. White accounted for most of the subjects in all treatment groups. The age, weight and BMI of all subjects fell within the inclusion criteria defined in the protocol.

| **Table 11: Subject demographics at baseline** | | |
|---|---|---|
| | Placebo | Total FR104 |
| Safety Population | 18 | 47 |
| Gender, n (%) | | |
| Male | 12 (67) | 26 (55) |
| Female | 6 | 21 |
| Age (years) | | |
| Mean (SD) | 52.7 (9.28) | 52.1 (7.88) |
| Median | 56.7 | 55.5 |
| Range | 20; 60 | 22; 60 |
| Race, n (%) | | |
| White | 17 (94) | 46 (98) |
| Asian | 1 (6) | 0 |
| Black/African | 0 | 1 (2) |
| American | | |
| BMI (kg/m²) | | |
| Mean (SD) | 25.04 (2.25) | 25.64 (2.196) |
| Median | 24.88 | 25.78 |
| Range | 20.2; 28.8 | 19.9; 29.7 |

### Pharmacokinetics

Lower doses of FR104 at 0.005, 0.020 and 0.050 mg/kg resulted in insufficient measurable concentrations for reliable PK parameter estimation. Figure 5A illustrates the FR104 concentration-time data for the remaining dose groups. In Cohort A (groups 1-4, no KLH immunization, FR104 0.005 to 0.500 mg/kg doses), the pharmacokinetics of FR104 were approximately linear from the 0.200 mg/kg dose with a t_{1/2} ranging from 146 (0.200 mg/kg) to 182 hours (0.500 mg/kg). In cohort B (groups 7-9bis, KLH immunization), as in cohort A, the pharmacokinetics of FR104 were approximately linear from the 0.200 mg/kg dose with a t_{1/2} ranging from 150 (0.200 mg/kg) to 210 hours (1.500 mg/kg). Mean FR104 Cmax ranged from 117 to 37700 ng/ml following doses 0.004-1.5 mg/Kg and AUCinf ranged from 0.705 to 7.010 mg/ml following doses 0.2-1.5 mg/Kg.

The PK of FR104 were also evaluated after two infusions of FR104 at 0.200 and 0.500 mg/kg given 28 days apart (Part 2). The pharmacokinetics were approximately linear with a t_{1/2} ranging from 169 to 203 hours. The PK parameters were similar after FR104 0.200 mg/kg infusion at Day 1 and Day 29. Accumulation of serum concentrations was observed after infusion of FR104 0.500 mg/kg, with an increase of about 20% in dose-normalized AUC0-28d and AUCinf.

### Pharmacodynamics

After infusion of FR104, a dose-dependent CD28 RO on the T cells in peripheral blood was observed (Figure 5B). The CD28 receptors were saturated at the first sampling time point (0.5 hours) after infusion of FR104 at doses of 0.020 mg/kg and higher. At Day 29, RO was still above 50% after infusion of FR104 at 0.500 mg/kg and 1.500 mg/kg doses. The CD28 RO returned to 50% in a dose dependent manner, by Day 15 (0.020 mg/kg) to Day 85 (1.500 mg/kg). The CD28 RO remained above 50% when the FR104 serum concentrations were just above the lower limit of quantification (<100 ng/mL), and above 80% when the FR104 serum concentrations were above 200 ng/mL. This suggests that FR104 has a high affinity to its receptor in vivo and that even low concentrations of FR104 may be pharmacologically active. After infusion of the second dose at 0.200 mg/Kg, receptors became saturated again, which resulted in a saturation above 50% lasting for more than 60 days. Two doses of 0.500 mg/kg given 28 days apart resulted in 100% saturation of the receptors for more than 60 days.

There was no significant change in the total lymphocyte count and lymphocyte subsets, including naive T cells, memory T cells and Treg cells, caused by FR104 at any dose and any dose regimen and the levels of these subsets did not significantly change in any group during the course of the study. The expression level of CD28 by lymphocyte subsets assessed by measuring MFI was unaffected either (Figure 6).

Owing to the cytokine release that previously occurred after administration in man of superagonist or divalent antagonist anti-CD28 mAbs, potential cytokine release has been closely followed up. No elevation of cytokines was observed in the serum of any volunteer. Only background levels have been recorded, which have been considered as non-clinically relevant (Figure 7eFigure 8A). Owing to this variability, the assay only picked up a significant inhibition of inducible IL-2 synthesis after administration of FR104 at doses above 0.100 mg/kg on the 2 and 96 hour time points but not at other time points (Figure 8B). SEB + LPS-induced synthesis of INFγ and IL-8, cytokines also secreted by non-T cells, was not modulated by treatment with FR104 in any group.

### Control of anti-KLH antibodies

The effect of FR104 on the response to KLH challenge was evaluated by measuring anti-KLH antibodies. The formation of anti-KLH antibodies significantly delayed and of a lower extent with increasing doses of FR104. After a single dose of 0.020 mg/kg, a reduction in the production of anti-KLH antibodies was already visible on Day 15 post-KLH immunization, which reached an average of 50% of the placebo group on Day 29. The anti-KLH response was maintained at that level in comparison with the placebo group until the last day of observation (Day 85). After the single dose of 0.200 mg/kg, the average reduction in the production of anti-KLH antibodies was of approx. 85% on days 15 and 29, and of 70% and 60% on days 57 and 85, respectively, as compared with the response of the placebo group. After the single dose of 0.500 mg/kg, the average reduction in the production of anti-KLH antibodies was >90% on Days 15 and 29, and approx. of 80% and 75% on Days 57 and 85, respectively. At the highest dose level (FR104 1.500 mg/kg), the formation of anti-KLH antibodies was essentially suppressed until Day 57. On Day 85 and 113, however, some response (approx. 75% reduction) was recorded (Figure 9).

### Immunogenicity

In Part 1 Cohort A (no KLH immunization), 0 (0%), 2 (50%), 3 (60.0%) and 1 (20.0%) subjects were reported as positive with antibodies against FR104 (ADAs) at the last sample (Day 85) following an infusion of FR104 at 0.005 mg/kg, 0.050 mg/kg, 0.200 mg/kg and 0.500 mg/kg, respectively. In Part I Cohort B (KLH immunization), 1 (20%), 3 (60.0%), 4 (80.0%) and 2 (40.0%) subjects had antibodies against FR104 at the last sample after FR104 0.020 mg/kg, 0.200 mg/kg, 0.500 mg/kg (Day 85), and 1.500 mg/kg (Day 113) infusion, respectively. In Part 2 (repeat doses), 3 subjects (60.0%) had antibodies against FR104 after both FR104 0.200 mg/kg and 0.500 mg/kg infusion at the last sample (Day 113). The earliest anti-FR104 antibodies were detected from Day 29 in Part 1 (Cohorts A and B) and from Day 57 in Part 2. Approximately half of the ADA+ subjects (10/22) were of relatively low titers (<30). They did not induce AEs nor modify cytokine release. Given that anti-FR104 antibodies when present appeared only after the disappearance of FR104 in blood in all subjects but one, no conclusion can be made on their potential impact on PK.

### Safety

No deaths nor SAEs occurred during the study except for one subject who was reported with the SAE nephrolithiasis that was considered not related to the study drug by the Investigator after FR104 0.500 mg/kg infusion and KLH challenge. None of the subjects discontinued the study drug or the study because of a treatment-emergent adverse event (TEAE). One subject in Part 2 did not receive the second dose of FR104 0.500 mg/kg on Day 29 due to receiving prohibited medication (ibuprofen, paracetamol and tramadol) for the AE myalgia. The subject was not withdrawn from the study and completed all study visits as per protocol. All reported TEAEs were of mild or moderate severity (Table 12). TEAEs considered to be at least possibly related to the study drug by the Investigator were reported in 5 of the 10 subjects who reported TEAEs after FR104 infusion and in none of the subjects after placebo infusion. By preferred term, the most frequently reported TEAEs after FR104 infusion were back pain, headache, and vomiting. Possibly treatment-related events after FR104 infusion were headache, vomiting, aphtous stomatitis, oral herpes, dry mouth, nausea, nasopharyngitis, fatigue, gingivitis, dysgeusia, diarrhea, vision blurred and influenza-like illness.

No clinically relevant or consistent changes in median values for laboratory, virology, vital signs, oxygen saturation and ECG parameters were observed after administration of the study drug in any volunteer.

Post-baseline EBV viral load results were negative or weak positive (except for 1 subject) and post-baseline EBV Capsid IgM antibody results were negative or equivocal for all subjects in the study (Figure 10). EBV capsid and nuclear antigen IgG antibodies were positive in all subjects (since it belonged to the inclusion criteria) and maintained in their baseline level throughout the study (data not shown). One subject receiving a 2 IV doses of placebo had a positive EBV viral load result on Day 15 with a viral load of log 3.83 IU/m, a value which would be considered high enough to be of clinical concern in a transplant setting, for example. At the next time point on Day 29, the test was negative. Later assessments were negative on Day 43, positive with viral load of 2.7 log IU/mL on Day 57, negative on Day 85 and positive on Day 113 with viral load <2.5 log IU/mL.

During the study, a QTcF interval >450 ms and a QTcF change from baseline of>30 ms were reported in 1 subject in Cohort B after placebo infusion and KLH challenge. In Part 2, a QTcF interval >450 ms was reported in 1 subject after FR104 0.200 mg/kg infusion, a QTcF change from baseline of >60 ms in 1 subject after FR104 0.200 mg/kg and a QTcF change from baseline of>30 ms was reported in 1 and 3 subjects after FR104 0.200 mg/kg and FR104 0.500 mg/kg infusion, respectively. No clinically significant vital signs, pulse oximetry or ECG abnormalities were observed in Part 1 (Cohort A and B) and Part 2.

No physical examination abnormalities were reported in during the study except for 2 subjects in Cohort A and 3 subject in Part 2. For these subjects, the abnormality was considered clinically significant and the observation was reported as TEAE: nasopharyngitis and conjunctivitis for the subject in Cohort A and upper respiratory tract infection, myalgia, and gout, for the subjects in Part 2.

**Table 12: A summary of TEAEs per treatment in Part 1 (Cohort A and B) and Part 2.**

| **Part 1, Cohort A** | | | | | | |
|---|---|---|---|---|---|---|
| | Placebo | FR104 0.005 mg/kg | FR104 0.050 mg/kg | FR104 0.200 mg/kg | FR104 0.500 mg/kg | FR104 All Subjects |
| Number of subjects with at least one: | | | | | | |
| TEAE | 2 (33.3) | 1 (33.3) | 3 (75.0) | 4 (80.0) | 2 (40.0) | 10 (58.8) |
| SAE | 0 | 0 | 0 | 0 | 0 | 0 |
| TEAE leading to death | 0 | 0 | 0 | 0 | 0 | 0 |
| Mild TEAE (worst severity) | 1 (16.7) | 0 | 3 (75.0) | 1 (20.0) | 0 | 4 (23.5) |
| Moderate TEAE (worst severity) | 1 (16.7) | 1 (33.3) | 0 | 3 (60.0) | 2 (40.0) | 6 (35.3) |
| Severe TEAE (worst severity) | 0 | 0 | 0 | 0 | 0 | 0 |
| TEAE for which the study drug was discontinued | 0 | 0 | 0 | 0 | 0 | 0 |
| At least possibly treatment-related TEAE | 0 | 0 | 1 (25.0) | 3 (60.0) | 1 (20.0) | 5 (29.4) |

| **Part 1, Cohort B** | | | | | | |
|---|---|---|---|---|---|---|
| | Placebo | FR104 0.020 mg/kg | FR104 0.200 mg/kg | FR104 0.500 mg/kg | FR104 1.500 mg/kg | FR104 All Subjects |
| Number of subjects with at least one: | | | | | | |
| TEAE | 7 (87.5) | 3 (60.0) | 3 (60.0) | 3 (60.0) | 1 (20.0) | 10 (50.0) |
| SAE | 0 | 0 | 0 | 1 (20.0) | 0 | 1 (5.0) |
| TEAE leading to death | 0 | 0 | 0 | 0 | 0 | 0 |
| Mild TEAE (worst severity) | 6 (75.0) | 2 (40.0) | 2 (40.0) | 1 (20.0) | 1 (20.0) | 6 (30.0) |
| Moderate TEAE (worst severity) | 1 (12.5) | 1 (20.0) | 1 (20.0) | 2 (40.0) | 0 | 4 (20.0) |
| Severe TEAE (worst severity) | 0 | 0 | 0 | 0 | 0 | 0 |
| TEAE for which the study drug was discontinued | 0 | 0 | 0 | 0 | 0 | 0 |
| At least possibly treatment-related TEAE | 2 (25.0) | 1 (20.0) | 1 (20.0) | 1 (20.0) | 0 | 3 (15.0) |

| **Part 2** | | | | | | |
|---|---|---|---|---|---|---|
| | Placebo | FR104 0.200 mg/kg | | FR104 0.500 mg/kg | FR104 All Subjects | |
| Number of subjects with at least one: | | | | | | |
| TEAE | 3 (75.0) | 5 (100.0) | | 4 (80.0) | 9 (90.0) | |
| SAE | 0 | 0 | | 0 | 0 | |
| TEAE leading to death | 0 | 0 | | 0 | 0 | |
| Mild TEAE (worst severity) | 2 (50.0) | 2 (40.0) | | 3 (60.0) | 5 (50.0) | |
| Moderate TEAE (worst severity) | 1 (25.0) | 3 (60.0) | | 1 (20.0) | 4 (40.0) | |
| Severe TEAE (worst severity) | 0 | 0 | | 0 | 0 | |
| TEAE for which the study drug was discontinued | 0 | 0 | | 0 | 0 | |
| At least possibly treatment-related TEAE | 1 (25.0) | 2 (40.0) | | 3 (60.0) | 5 (50.0) | |

### DISCUSSION

This trial represents the first administration to humans of FR104, a humanized pegylated Fab' antibody fragment antagonist of CD28. FR104 was well tolerated at all doses, with the most common treatment-emergent AE being headache. FR104 administration was not associated with cytokine release syndrome or activation of T cells. In previous attempts to target CD28 using anti-CD28 monoclonal antibodies (TGN1412, (Suntharalingam et al), FK734 (Shiao et al)) clinical development was complicated by induced cytokine release. As FR104 was not associated with such complications, targeting CD28 with monovalent antagonist antibodies remains an attractive therapeutic strategy for costimulation blockade.

Cytokine assessment in serum in man demonstrated complete absence of agonist or superagonist activity of FR104 at any dose, even after repeat dose administration. Background or close to background levels of IFNg, TNFa and IL-8 in some subjects recorded at baseline or after FR104 administration (Figure 7) are far below those measured after administration of TGN1412, which peaked at 5000 pg/ml for these two cytokines (Suntharalingam et al). Antidrug antibodies (ADA) when present became apparent in most subject only after FR104 elimination from the plasma. Therefore, it is difficult to estimate the effect of ADA on PK and PD parameters and especially on cytokines. In one subject however (Part 2, dose 0.2 mg/kg MAD), ADA became apparent while CD28 RO was still measurable. In this subject, there was no cytokine release either, confirming the preclinical observation that FR104 in the presence of ADA do not recapitulated the agonistic properties of anti-CD28 antibodies in their IgG format. Of note, the relatively high incidence of immunogenicity for FR104 is in a similar range to what has been reported for authorized biological Cimzia® (Certolizumab Pegol), a pegylated Fab' antibody fragment and some other biologics such as Remicade and Humira; CIMZIA clinical pharmacology BLA 125160/0). FR104 did not alter total lymphocyte counts or lymphocyte subsets. T cell phenotype was also unmodified. Given that CD28 may also regulate migration of primed T cells to target tissue, these data together with absence of cytokine release and absence of target T cell activation reinforce the purely antagonist activity of FR104.

The PK of FR104 can be considered as approximately linear at doses ≥0.200 mg/kg. Only slight variations in t1/2 and in AUCinf and related parameters were observed between groups (SAD and MAD) for the same dose.

### REFERENCES

Bowen, M., Armstrong, N., and Maa, Y.-F. (2012). Investigating highconcentration monoclonal antibody powder suspension in nonaqueous suspension vehicles for subcutaneous injection. J. Pharm. Sci. 101, 4433-4443.
Findlay L, Eastwood D, Stebbings R, Sharp G, Mistry Y, Ball C, et al. Improved in vitro methods to predict the in vivo toxicity in man of therapeutic monoclonal antibodies including TGN1412. J Immunol Methods. 2010;352:1-12.
Haanstra KG., Dijkman K., Bashir N., et al. Selective Blockade of CD28-Mediated T Cell Costimulation Protects Rhesus Monkeys against Acute Fatal Experimental Autoimmune Encephalomyelitis. J Immunol. 2015 Jan 14. pii: 1402563. [Epub ahead of print].
Haley PJ. Small molecule immunomodulatory drugs: challenges and approaches for balancing efficacy with toxicity. Toxicol Pathol. 2012;40(2):261-266.
Jackisch, C., Müller, V., Maintz, C., Hell, S., and Ataseven, B. (2014). Subcutaneous Administration of Monoclonal Antibodies in Oncology. Geburtshilfe Frauenheilkd. 74, 343-349.
Kean L. et al, T cell Costimulation Blockade in Transplantation. Oral communication at the Nantes Actualités Transplantation Meeting, June 5-6, 2014, Nantes, France
Melichar, B., Študentová, H., Kalábová, H., and Vitásková, D. (2014). Role of subcutaneous formulation of trastuzumab in the treatment of patients with HER2-positive breast cancer. Immunotherapy 6, 811-819.
Ohresser M, Olive D, Vanhove B, Watier H. Risk in drug trials. Lancet. 2006 368(9554):2205-6.
Poirier, N., Dilek, N., Mary, C., et al. FR104, an Antagonist Anti-CD28 Monovalent Fab' Antibody, Prevents Alloimmunization and Allows Calcineurin Inhibitor Minimization in Nonhuman Primate Renal Allograft. Am J Transplant. 2015 (1):88-100.
Poirier, N., Mary, C., Dilek, N. et al. Preclinical efficacy and immunological safety of FR104, an antagonist anti-CD28 monovalent Fab' antibody. Am J Transplant. 2012 (10):2630-40.
Poirier, N., Mary, C., Le Bas-Bernadet, S., N. et al. Advantages of Papio anubis for preclinical testing of immunotoxicity of candidate therapeutic antagonist antibodies targeting CD28. MAbs. 2014 (3):697-707.
Shiao SL, McNiff JM, Masunaga T, Tamura K, Kubo K, and Pober JS. Immunomodulatory properties of FK734, a humanized anti-CD28 monoclonal antibody with agonistic and antagonistic activities. Transplantation. 2007;83(3):304-13.
Shpilberg, O., and Jackisch, C. (2013). Subcutaneous administration of rituximab (MabThera) and trastuzumab (Herceptin) using hyaluronidase. Br. J. Cancer 109, 1556-1561.
Stebbings R, Findlay L, Edwards C, Eastwood D, Bird C, North D, et al. 'Cytokine storm' in the phase I trial of monoclonal antibody TGN1412: better understanding the causes to improve pre-clinical testing of immunotherapeutics. J Immunol. 2007; 179:3325-3331.
Suntharalingam G, Perry MR, Ward S, Brett SJ, Castello-Cortes A, Brunner MD, and Panoskaltsis N. Cytokine storm in a phase 1 trial of the anti-CD28 monoclonal antibody TGN1412. N Engl J Med. 2006;355(10):1018-28.
Vierboom et al., Clinical efficacy of a new CD28-targeting antagonist of T cell co-stimulation in a non-human primate model of collagen-induced arthritis. Clin Exp Immunol. 2015.
Weinblatt, M.E., Schiff, M., Valente, R., van der Heijde, D., Citera, G., Zhao, C., Maldonado, M., and Fleischmann, R. (2013). Head-to-head comparison of subcutaneous abatacept versus adalimumab for rheumatoid arthritis: findings of a phase IIIb, multinational, prospective, randomized study. Arthritis Rheum. 65, 28-38.

### Further numbered embodiments of the invention:

1. An anti-CD28 Fab' antibody fragment consisting of a heterodimer of (i) a first protein of SEQ ID NO: 1, which is pegylated at its C-terminus, and (ii) a second protein of SEQ ID NO: 2, for use in the treatment of any condition susceptible of being improved or prevented by inhibiting a T cell immune response, wherein a therapeutically effective amount of said anti-CD28 Fab' antibody fragment is administered to a human subject in need thereof and wherein the therapeutically effective amount of the anti-CD28 Fab' antibody fragment is between 0.05 and 1.5 mg/kg body weight at a dosing schedule of once per week, once every two weeks, once every three weeks, once every four weeks, once every five weeks or once every 6 weeks, once every 7 weeks, once every 8 weeks or once every more than 8 weeks.
2. The anti-CD28 Fab' antibody fragment of embodiment 1, for the use of embodiment 1, wherein the therapeutically effective amount is between 0.05 and less than 0.5 mg/kg body weight, administered at a dosing schedule of once per week, once every two weeks, once every three weeks, once every four weeks, once every five weeks or once every 6 weeks.
3. The anti-CD28 Fab' antibody fragment of embodiment 1, for the use of embodiment 1 or embodiment 2, wherein the therapeutically effective amount is between 0.05 and 0.2 mg/kg body weight administered at a dosing schedule of once per week, once every two weeks, once every three weeks, once every four weeks or once every five weeks.
4. The anti-CD28 Fab' antibody fragment of embodiment 1, for the use of embodiment 1, wherein the therapeutically effective amount is between 0.5 and 1.5 mg/kg body weight, administered at a dosing schedule of from once every at least four weeks for 0.5 mg/kgto once every at least 8 weeks for 1 mg/kg and once every more than 8 weeks for doses above 1 mg/kg.
5. The anti-CD28 Fab' antibody fragment of embodiment 1, for the use of any of embodiments 1 to 4, wherein the therapeutically effective amount of an anti-CD28 Fab' antibody fragment induces at least 80% CD28 receptor occupancy over the period of time between two administrations of said anti-CD28 Fab' antibody fragment.
6. The anti-CD28 Fab' antibody fragment of embodiment 1, for the use of any of embodiments 1 to 5, wherein the condition susceptible of being improved or prevented by inhibiting a T cell immune response is a transplanted organ, tissue or cell dysfunction, a T-lymphocyte-mediated autoimmune disease, atherosclerosis or an inflammatory disease in said subject.
7. The anti-CD28 Fab' antibody fragment of embodiment 1, for the use of any of embodiments 1 to 6, wherein the condition susceptible of being improved or prevented by inhibiting a T cell immune response is a kidney transplant rejection, a chronic allograft vasculopathy, a graft-versus-host disease, an autoimmune encephalomyelitis, a psoriasis, a rheumatoid arthritis, a multiple sclerosis, a Crohn' s disease, an ulcerative colitis, atherosclerosis, a type 1 diabetes or a type IV hypersensitivity.
8. The anti-CD28 Fab' antibody fragment of embodiment 1, for the use of any of embodiments 1 to 7, wherein the anti-CD28 Fab' antibody fragment is administered to said subject intravenously, subcutaneously, intramuscularly, topically via intrathecal injection.
9. A pharmaceutical composition for inhibiting a T cell immune response in a human subject in need thereof, comprising an anti-CD28 Fab' antibody fragment which is a heterodimer of (i) a first protein of SEQ ID NO: 1, pegylated at its C-terminus, and (ii) a second protein of SEQ ID NO: 2 in an amount comprised between 3 and 120 mg, together with one or more pharmaceutically acceptable excipients.
10. The pharmaceutical composition of embodiment 9, which comprises less than 35 mg, preferably between 3 and 14 mg of said anti-CD28 Fab' antibody fragment.
11. The pharmaceutical composition of embodiment 9 or embodiment 10, which is suitable for intravenous, subcutaneous, intramuscular, topical or intrathecal administration.
12. The pharmaceutical composition of any of embodiments 9 to 11, which comprises recombinant human hyaluronidase.
13. The pharmaceutical composition of any of embodiments 9, 11 or 12, which comprises between 35 mg and 120 mg of said anti-CD28 Fab' antibody fragment.
14. A kit of parts comprising monthly doses of an anti-CD28 Fab' antibody fragment, wherein each dose comprises an amount of 3 to 120 mg of an anti-CD28 Fab' antibody fragment which is a heterodimer of (i) a first protein of SEQ ID NO: 1, pegylated at its C-terminus, and (ii) a second protein of SEQ ID NO: 2.
15. A syringe comprising the pharmaceutical composition of any of embodiments 9 to 13.

## Claims

1. An anti-CD28 Fab' antibody fragment consisting of a heterodimer of (i) a first protein of SEQ ID NO: 1, which is pegylated at its C-terminus, and (ii) a second protein of SEQ ID NO: 2, for use in the treatment of any condition susceptible of being improved or prevented by inhibiting a T cell immune response, wherein a therapeutically effective amount of said anti-CD28 Fab' antibody fragment is administered to a human subject in need thereof and wherein the therapeutically effective amount of the anti-CD28 Fab' antibody fragment is between 0.05 and 1.5 mg/kg body weight at a dosing schedule of once per week, once every two weeks, once every three weeks, once every four weeks, once every five weeks or once every 6 weeks, once every 7 weeks, once every 8 weeks or once every more than 8 weeks.

2. The anti-CD28 Fab' antibody fragment of claim 1, for the use of claim 1, wherein the therapeutically effective amount is between 0.05 and less than 0.5 mg/kg body weight, administered at a dosing schedule of once per week, once every two weeks, once every three weeks, once every four weeks, once every five weeks or once every 6 weeks.

3. The anti-CD28 Fab' antibody fragment of claim 1, for the use of claim 1 or claim 2, wherein the therapeutically effective amount is between 0.05 and 0.2 mg/kg body weight administered at a dosing schedule of once per week, once every two weeks, once every three weeks, once every four weeks or once every five weeks.

4. The anti-CD28 Fab' antibody fragment of claim 1, for the use of claim 1, wherein the therapeutically effective amount is between 0.5 and 1.5 mg/kg body weight, administered at a dosing schedule of from once every at least four weeks for 0.5 mg/kgto once every at least 8 weeks for 1 mg/kg and once every more than 8 weeks for doses above 1 mg/kg.

5. The anti-CD28 Fab' antibody fragment of claim 1, for the use of any of claims 1 to 4, wherein the therapeutically effective amount of an anti-CD28 Fab' antibody fragment induces at least 80% CD28 receptor occupancy over the period of time between two administrations of said anti-CD28 Fab' antibody fragment.

6. The anti-CD28 Fab' antibody fragment of claim 1, for the use of claim 1, wherein the therapeutically effective amount is 0.5 ± 10% mg/kg body weight administered at a dosing schedule of once every two weeks.

7. The anti-CD28 Fab' antibody fragment of claim 1, for the use of claim 1, wherein the therapeutically effective amount is 1.5 mg/kg body weight administered at a dosing schedule of once every four weeks.

8. The anti-CD28 Fab' antibody fragment of claim 1, for the use of any of claims 1 to 7, wherein the condition susceptible of being improved or prevented by inhibiting a T cell immune response is a transplanted organ, tissue or cell dysfunction, a T-lymphocyte-mediated autoimmune disease, atherosclerosis or an inflammatory disease or type IV hypersensitivity in said subject.

9. The anti-CD28 Fab' antibody fragment of claim 1, for the use of any of claims 1 to 8, wherein the condition susceptible of being improved or prevented by inhibiting a T cell immune response is a transplanted tissue rejection, a chronic allograft vasculopathy, a graft-versus-host disease, an autoimmune encephalomyelitis, a psoriasis, a rheumatoid arthritis, a multiple sclerosis, a Crohn's disease, an ulcerative colitis, atherosclerosis, a type 1 diabetes or a type IV hypersensitivity.

10. The anti-CD28 Fab' antibody fragment of claim 1, for the use of any of claims 1 to 9, wherein the anti-CD28 Fab' antibody fragment is administered to said subject intravenously, subcutaneously, intramuscularly, topically via intrathecal injection.

11. A pharmaceutical composition for inhibiting a T cell immune response in a human subject in need thereof, comprising an anti-CD28 Fab' antibody fragment which is a heterodimer of (i) a first protein of SEQ ID NO: 1, pegylated at its C-terminus, and (ii) a second protein of SEQ ID NO: 2 in an amount comprised between 3 and 120 mg, together with one or more pharmaceutically acceptable excipients.

12. The pharmaceutical composition of claim 11, which comprises less than 35 mg, preferably between 3 and 14 mg of said anti-CD28 Fab' antibody fragment.

13. The pharmaceutical composition of claim 11 or claim 12, which is suitable for intravenous, subcutaneous, intramuscular, topical or intrathecal administration.

14. A kit of parts comprising monthly doses of an anti-CD28 Fab' antibody fragment, wherein each dose comprises an amount of 3 to 120 mg of an anti-CD28 Fab' antibody fragment which is a heterodimer of (i) a first protein of SEQ ID NO: 1, pegylated at its C-terminus, and (ii) a second protein of SEQ ID NO: 2.

15. A syringe comprising the pharmaceutical composition of any of claims 11 to 13.


SEQUENCE LISTING

<110>  OSE IMMUNOTHERAPEUTICS

<120>  ANTI-CD28 HUMANIZED ANTIBODIES FORMULATED FOR ADMINISTRATION TO
       HUMANS

<130>

<150>  EP 15200281.2
<151>  2015-12-15

<150>  EP 16306537.8
<151>  2016-11-22

<160>  4

<170>  PatentIn version 3.5

<210>  1
<211>  232
<212>  PRT
<213>  Artificial Sequence

<220>
<223>  VH-hCH1


<220>
<221>  MISC_FEATURE
<222>  (1)..(1)
<223>  Xaa= Gln or None

<400>  1

Xaa Val Gln Leu Gln Gln Ser Gly Ala Glu Leu Lys Lys Pro Gly Ala
1               5                   10                  15


Ser Val Lys Val Ser Cys Lys Ala Ser Gly Tyr Thr Phe Thr Glu Tyr
            20                  25                  30


Ile Ile His Trp Ile Lys Leu Arg Ser Gly Gln Gly Leu Glu Trp Ile
        35                  40                  45


Gly Trp Phe Tyr Pro Gly Ser Asn Asp Ile Gln Tyr Asn Ala Gln Phe
    50                  55                  60


Lys Gly Lys Ala Thr Leu Thr Ala Asp Lys Ser Ser Ser Thr Val Tyr
65                  70                  75                  80


Met Glu Leu Thr Gly Leu Thr Pro Glu Asp Ser Ala Val Tyr Phe Cys
                85                  90                  95


Ala Arg Arg Asp Asp Phe Ser Gly Tyr Asp Ala Leu Pro Tyr Trp Gly
            100                 105                 110

Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Ala Ala
225                 230

<210>  2
<211>  214
<212>  PRT
<213>  Artificial Sequence

<220>
<223>  VL-hCkappa

<220>
<221>  MISC_FEATURE
<222>  (96)..(96)
<223>  Xaa = Cys or Ala or Asn

<400>  2

Asp Ile Gln Met Thr Gln Ser Pro Ser Ser Leu Ser Ala Ser Val Gly
1               5                   10                  15

Asp Arg Val Thr Ile Thr Cys Lys Thr Asn Glu Asn Ile Tyr Ser Asn
            20                  25                  30

Leu Ala Trp Tyr Gln Gln Lys Asp Gly Lys Ser Pro Gln Leu Leu Ile
        35                  40                  45

Tyr Ala Ala Thr His Leu Val Glu Gly Val Pro Ser Arg Phe Ser Gly
    50                  55                  60

Ser Gly Ser Gly Thr Gln Tyr Ser Leu Thr Ile Ser Ser Leu Gln Pro
65                  70                  75                  80

Glu Asp Phe Gly Asn Tyr Tyr Cys Gln His Phe Trp Gly Thr Pro Xaa
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  3
<211>  232
<212>  PRT
<213>  Artificial Sequence

<220>
<223>  VH-hCH1

<400>  3

Gln Val Gln Leu Gln Gln Ser Gly Ala Glu Leu Lys Lys Pro Gly Ala
1               5                   10                  15

Ser Val Lys Val Ser Cys Lys Ala Ser Gly Tyr Thr Phe Thr Glu Tyr
            20                  25                  30

Ile Ile His Trp Ile Lys Leu Arg Ser Gly Gln Gly Leu Glu Trp Ile
        35                  40                  45

Gly Trp Phe Tyr Pro Gly Ser Asn Asp Ile Gln Tyr Asn Ala Gln Phe
    50                  55                  60

Lys Gly Lys Ala Thr Leu Thr Ala Asp Lys Ser Ser Ser Thr Val Tyr
65                  70                  75                  80

Met Glu Leu Thr Gly Leu Thr Pro Glu Asp Ser Ala Val Tyr Phe Cys
                85                  90                  95

Ala Arg Arg Asp Asp Phe Ser Gly Tyr Asp Ala Leu Pro Tyr Trp Gly
            100                 105                 110

Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Ala Ala
225                 230

<210>  4
<211>  214
<212>  PRT
<213>  Artificial Sequence

<220>
<223>  VL-hCkappa

<400>  4

Asp Ile Gln Met Thr Gln Ser Pro Ser Ser Leu Ser Ala Ser Val Gly
1               5                   10                  15

Asp Arg Val Thr Ile Thr Cys Lys Thr Asn Glu Asn Ile Tyr Ser Asn

            20                  25                  30

Leu Ala Trp Tyr Gln Gln Lys Asp Gly Lys Ser Pro Gln Leu Leu Ile
        35                  40                  45

Tyr Ala Ala Thr His Leu Val Glu Gly Val Pro Ser Arg Phe Ser Gly
    50                  55                  60

Ser Gly Ser Gly Thr Gln Tyr Ser Leu Thr Ile Ser Ser Leu Gln Pro
65                  70                  75                  80

Glu Asp Phe Gly Asn Tyr Tyr Cys Gln His Phe Trp Gly Thr Pro Cys
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210